(19) Europäisches Patentamt
European Patent Office
Office européen des brevets

(11) EP 3 009 438 A1

(12) EUROPEAN PATENT APPLICATION
published in accordance with Art. 153(4) EPC

(43) Date of publication:
**20.04.2016 Bulletin 2016/16**

(21) Application number: **14810399.7**

(22) Date of filing: **09.06.2014**

(51) Int Cl.:
***C07D 491/147*** *(2006.01)* ***C09K 11/06*** *(2006.01)*
***H01L 51/50*** *(2006.01)*

(86) International application number:
**PCT/JP2014/065215**

(87) International publication number:
**WO 2014/199943 (18.12.2014 Gazette 2014/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.06.2013 JP 2013125118**

(71) Applicant: **Hodogaya Chemical Co., Ltd.**
**Tokyo 104-0028 (JP)**

(72) Inventors:
- **YOKOYAMA, Norimasa**
**Tokyo 104-0028 (JP)**
- **KANDA, Daizou**
**Tokyo 104-0028 (JP)**
- **HAYASHI, Shuichi**
**Tokyo 104-0028 (JP)**

(74) Representative: **Müller-Boré & Partner**
**Patentanwälte PartG mbB**
**Friedenheimer Brücke 21**
**80639 München (DE)**

# (54) DICARBAZOLE DERIVATIVE AND ORGANIC ELECTROLUMINESCENT ELEMENT

(57) Dicarbazole derivatives represented by the following general formula (1),

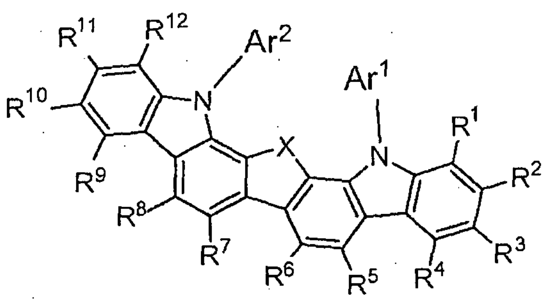

(1)

wherein X is an oxygen atom or a sulfur atom, $Ar^1$ and $Ar^2$ are aromatic hydrocarbon groups or aromatic heterocyclic groups, $R^1$ to $R^{12}$ are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, aryloxy groups or disubstituted amino groups having aromatic hydrocarbon groups or aromatic heterocyclic groups as substituents bonded to the nitrogen atom.

EP 3 009 438 A1

**Description**

Technical Field:

This invention relates to novel dicarbazole derivatives and, more specifically, to novel dicarbazole derivatives suited for an organic electroluminescent device that is a spontaneously luminous device and can be favorably used for various kinds of display devices.

Background Art:

An organic electroluminescent device (hereinafter often called organic EL device) is a spontaneously luminous device which features higher brightness and higher legibility than those of the liquid crystal devices enabling vivid display to be attained and has, therefore, been vigorously studied.

In 1987, C. W. Tang et al. of the Eastman Kodak Co. have developed a device of a layer-laminated structure comprising various kinds of materials to bear individual roles, and have put an organic EL device using organic materials into a practical use. The above organic EL device is constituted by laminating layers of a fluorescent body capable of transporting electrons and an aromatic amine compound capable of transporting holes. Upon injecting both electric charges into the layer of the fluorescent body to emit light, the device is capable of attaining a brightness of as high as 1000 cd/$m^2$ or more with a voltage of not higher than 10 V.

So far, very many improvements have been made to put the organic EL device to practical use. Namely, a high efficiency and a high durability have been achieved by fabricating an electric field luminous device that comprises an anode, a hole injection layer, a hole-transporting layer, a luminous layer, an electron-transporting layer, an electron injection layer and a cathode that are arranged in this order on a substrate more finely dividing their roles than ever before.

To further improve the luminous efficiency, attempts have been made to utilize triplet excitons, study has been forwarded to utilize a phosphorescent luminous body, and a device has been developed utilizing the luminance based on the thermally activated delayed fluorescence (TADF).

The luminous layer can also be prepared by doping an electron charge-transporting compound that is, usually, called host material with a florescent body or a phosphorescent luminous body.

Various properties such as efficiency and durability of the device are seriously affected by the selection of the organic materials used for the organic EL device.

In the organic EL device, the electric charges injected from the two electrodes recombine together in the luminous layer to emit light. Here, what is important is how efficiently to hand both electric charges, i.e., holes and electrons, over to the luminous layer. Upon improving the probability of recombination of holes and electrons by improving the hole injection property and by improving property for blocking the electrons injected through the cathode, and, further, confining the excitons formed in the luminous layer, it is allowed to attain a high luminous efficiency. Namely, the electron-transporting material plays an important role. Therefore, it has been desired to provide an electron-transporting material that has a high electron injection property, a large electron migration rate, a high electron-blocking property and a large durability against the electrons.

As for the life of the device, further, the heat resistance and amorphousness of the material also serve as important factors. The material having small heat resistance is subject to be thermally decomposed even at a low temperature due to the heat generated when the device is driven, and is deteriorated. The material having low amorphousness permits the thin film thereof to be crystallized in short periods of time and, therefore, the device to be deteriorated. Therefore, the material to be used must have large heat resistance and good amorphousness.

As the hole-transporting materials used for the organic EL devices, there have heretofore been known an N,N'-diphenyl-N,N'-di($\alpha$-naphthyl) benzidine (hereinafter abbreviated as NPD) and various aromatic amine derivatives. The NPD has a favorable hole-transporting capability but its glass transition point (Tg) that serves as an index of heat resistance is as low as 96°C permitting, therefore, the properties of the device to be deteriorated due to the crystallization thereof under high-temperature conditions. Further, it has been known that some of the aromatic amine derivatives have a hole mobility of as excellent as $10^{-3}$ $cm^2/Vs$ or more accompanied, however, by insufficient electron-blocking capability and, therefore, permitting part of the electrons to pass through the luminous layer making it difficult to improve the luminous efficiency. To further improve the efficiency, it has been urged to provide a material having higher electron-blocking capability, and having higher stability and heat resistance in the form of thin films.

As the compounds improving such properties as heat resistance, hole injection property and electron-blocking property, there have been proposed arylamine compounds (e. g. , compounds A and B) having substituted carbazole structures represented by the following formulas (e.g., see patent documents 1 and 2).

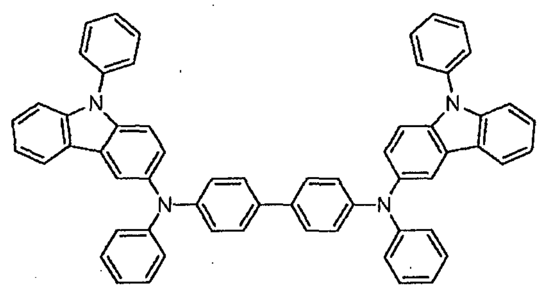

(Compound A)

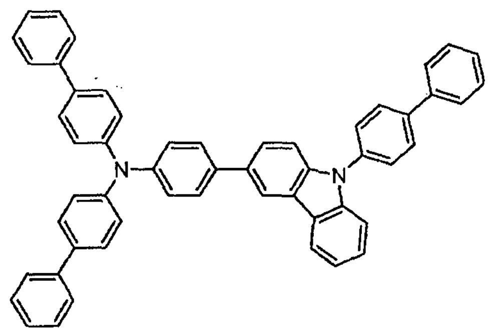

(Compound B)

The devices using these compounds as the hole injection layer or the hole-transporting layer exhibit improvements in the heat resistance and luminous efficiency which, however, are not still sufficient. Moreover, drivability with a low voltage and current efficiency are not sufficient, and amorphousness still involves a problem. Therefore, it has been urged to attain drivability with a further decreased voltage and higher luminous efficiency while improving amorphousness.

Prior Art Documents:

Patent Documents:

Patent document 1: JP-A-2006/151979
Patent document 2: WO2008/62636

Outline of the Invention:

Problems that the Invention is to Solve:

It is an object of the present invention to provide an organic compound having excellent hole injection/hole-transporting capability, electron-blocking capability, high stability in the formof a thin film and excellent heat stability, that can be used as a material for fabricating organic electroluminescent devices that feature high efficiency and large durability.
Another object of the present invention is to provide organic electroluminescent devices fabricated by using the above organic compound to feature high luminous efficiency and large durability.

Means for Solving the Problems:

In order to achieve the above objects, the present inventors have chemically synthesized the compounds having a furodicarbazole ring structure or a thienodicarbazole ring structure expecting that the carbazole structure would possess a hole injection/hole-transporting capability while the furodicarbazole ring structure or the thienodicarbazole ring structure would exhibit an electron-blocking capability and would possess a heat resistance and a stability in the form of a thin film. Further, the inventors have fabricated various organic EL devices using the above compounds on a trial basis, have keenly evaluated the properties of the devices, and have completed the present invention.
According to the present invention, there are provided dicarbazole derivatives represented by the following general formula (1),

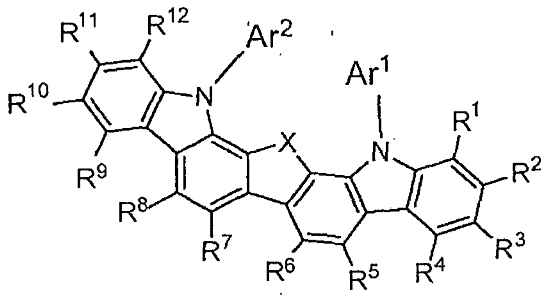

(1)

wherein,

X is an oxygen atom or a sulfur atom,
$Ar^1$ and $Ar^2$ are aromatic hydrocarbon groups or aromatic heterocyclic groups, and
$R^1$ to $R^{12}$ are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, aryloxy groups or disubstituted amino groups having aromatic hydrocarbon groups or aromatic heterocyclic groups as substituents bonded to the nitrogen atom, $R^1$ to $R^{12}$ may be singularly bonded or bonded to each other via a methylene group, an oxygen atom or a sulfur atom to form a ring.

Physical properties to be possessed by the organic compounds provided by the invention are (1) good hole injection property, (2) large hole mobility, (3) excellent electron-blocking capability, (4) stability in the form of a thin film, and (5) excellent heat resistance. Moreover, physical properties to be possessed by the organic electroluminescent device provided by the invention are (1) high luminous efficiency and power efficiency, (2) low luminance start voltage and (3) low practical driving voltage.
In the dicarbazole derivatives of the invention, it is desired that:

(1) In the above general formula (1), X is an oxygen atom and the dicarbazole derivatives have a furodicarbazole ring structure; and
(2) In the above general formula (1), X is a sulfur atom and the dicarbazole derivatives have a thienodicarbazole ring structure.

In these dicarbazole derivatives, further, it is desired that:

(3) In the above general formula (1), $R^1$, $R^2$, $R^4$ to $R^9$, $R^{11}$ and $R^{12}$ are hydrogen atoms or deuterium atoms;
(4) In the above general formula (1), $R^1$, $R^2$, $R^4$ to $R^9$, $R^{11}$ and $R^{12}$ are hydrogen atoms;
(5) In the above general formula (1), $Ar^1$ is an unsubstituted phenyl group;
(6) $Ar^1$ is an unsubstituted phenyl group, and $Ar^2$ is a group different from $Ar^1$; and
(7) $Ar^2$ is a phenyl group having a substituent.

The invention, further, provides an organic electroluminescent device comprising a pair of electrodes and at least one organic layer sandwiched therebetween, wherein the dicarbazole derivative is used as a material for constituting at least one organic layer.
In the organic EL device of the present invention, it is desired that the organic layer formed by using the dicarbazole derivative is a hole-transporting layer, an electron-blocking layer, a hole injection layer or a luminous layer.

Effects of the Invention:

As will be understood from the above general formula (1), the dicarbazole derivatives of the present invention have the furodicarbazole ring structure (X is an oxygen atom in the general formula (1)) or the thienodicarbazole ring structure (X is a sulfur atom in the general formula (1)) and, being related to such structures, have the following properties

(A) Good hole injection property;
(B) Large hole mobility;
(C) Excellent electron-blocking capability;

(D) Stability in the form of a thin film (excellent amorphousness); and
(E) Excellent heat resistance.

As described above, the dicarbazole derivatives of the invention remain stable in the form of a thin film and, besides, have excellent properties such as hole mobility and electron-blocking capability. Therefore, the dicarbazole derivatives can be favorably used for forming an organic layer between the electrodes of the organic EL device, and are capable of imparting the following properties to the organic EL device.

(F) High luminous efficiency and power efficiency;
(G) Low luminance start voltage;
(H) Low practical driving voltage; and
(I) Long service life (large durability).

For instance, the organic EL device has the hole injection layer and/or the hole-transporting layer formed by using the above dicarbazole derivatives and, therefore, has a high hole injection capability, a high hole mobility and a high electron-blocking power. Therefore, the excitons formed in the luminous layer can be confined therein and, besides, the holes and the electrons can be recombined at an increased probability bringing about, therefore, not only a high luminous efficiency but also a low driving voltage and, hence, improved durability.
Moreover, the dicarbazole derivatives of the invention feature excellent hole-transporting capability and a wide band gap. By using the derivatives as a host material to carry a fluorescence emission material or a phosphorous luminous body called dopant thereon to thereby form a luminous layer, therefore, it is made possible to obtain an organic EL device having a low driving voltage and improved luminous efficiency.

Brief Description of the Drawings:

[Fig. 1] is a $^{1}$H-NMR chart of a compound (compound 4) of Example 1.
[Fig. 2] is a $^{1}$H-NMR chart of a compound (compound 5) of Example 2.
[Fig. 3] is a $^{1}$H-NMR chart of a compound (compound 6) of Example 3.
[Fig. 4] is a $^{1}$H-NMR chart of a compound (compound 25) of Example 4.
[Fig. 5] is a $^{1}$H-NMR chart of a compound (compound 26) of Example 5.
[Fig. 6] is a $^{1}$H-NMR chart of a compound (compound 27) of Example 6.
[Fig. 7] is a view illustrating the structure of layers of an organic EL device.

Modes for Carrying Out the Invention:

The dicarbazole derivatives of the present invention are novel compounds and are represented by the following general formula (1).

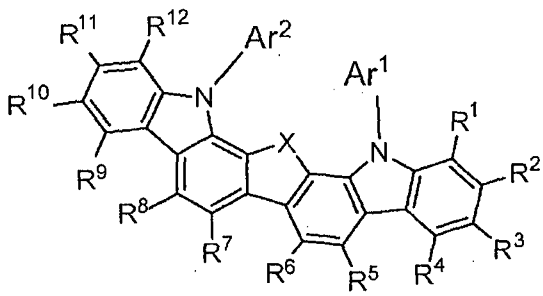

(1)

<X>

In the above general formula (1), X is an oxygen atom or a sulfur atom.
That is, if X is the oxygen atom in the general formula (1), the dicarbazole derivatives of the invention have the furodicarbazole ring structure. If X is the sulfur atom in the general formula (1), the dicarbazole derivatives of the invention have the thienodicarbazole ring structure.

<$Ar^1$ and $Ar^2$>

In the general formula (1), further, $Ar^1$ and $Ar^2$ are aromatic hydrocarbon groups or aromatic heterocyclic groups. The aromatic hydrocarbon groups and the aromatic heterocyclic groups may have a monocyclic structure or a condensed polycyclic structure.

As the aromatic groups (aromatic hydrocarbon groups and aromatic heterocyclic groups), there can be exemplified phenyl group, biphenylyl group, terphenylyl group, naphthyl group, anthryl group, phenanthryl group, fluorenylgroup, indenylgroup, pyrenyl group, perylenyl group, fluoranthenyl group, triphenylenyl group, pyridyl group, furyl group, pyrrolyl group, thienyl group, quinolyl group, isoquinolyl group, benzofuranyl group, benzothienyl group, indolyl group, carbazolyl group, benzoxazolyl group, benzothiazolyl group, quinoxalyl group, benzimidazolyl group, pyrazolyl group, dibenzofuranyl group, dibenzothienyl group and carbolynyl group.

In the invention, among the groups exemplified above, specifically preferred are aromatic hydrocarbon groups such as phenyl group, biphenyl group, fluorenyl group and triphenylenyl group; sulfur-containing aromatic heterocyclic groups such as thienyl group, benzothienyl group, benzothiazolyl group and dibenzothienyl group; oxygen-containing aromatic heterocyclic groups such as furyl group, benzofuranyl group, benzoxazolyl group and dibenzofuranyl group; and nitrogen-containing aromatic heterocyclic groups such as carbazolyl group. Among them, particularly preferred are phenyl group, biphenylyl group, fluorenyl group and N-phenylcarbazolyl group.

The above aromatic group may have a substituent. As the substituent, there can be exemplified deuterium atom; cyano group; nitro group; halogen atoms such as fluorine atom, chlorine atom, bromine atom and iodine atom; alkyl groups having 1 to 6 carbon atoms, such as methyl group, ethyl group, n-propyl group, isopropyl group, n-butyl group, isobutyl group, tert-butylgroup, n-pentyl group, isopentyl group, neopentyl group and n-hexyl group; alkyloxy groups having 1 to 6 carbon atoms, such as methyloxy group, ethyloxy group and propyloxy group; alkenyl groups such as allyl group; aryloxy groups such as phenyloxy group and tolyloxy group; arylalkyloxy groups such as benzyloxy group and phenetyloxy group; aromatic hydrocarbon groups such as phenyl group, biphenylyl group, terphenylyl group, naphthyl group, anthracenyl group, phenanthryl group, fluorenyl group, indenyl group, pyrenyl group, perylenyl group, fluoranthenyl group and triphenylenyl group; aromatic heterocyclic groups such as pyridyl group, thienyl group, furyl group, pyrrolyl group, quinolyl group, isoquinolyl group, benzofuranyl group, benzothienyl group, indolyl group, carbazolyl group, benzoxazolyl group, benzothiazolyl group, quinoxalyl group, benzimidazolyl group, pyrazolyl group, dibenzofuranyl group, dibenzothienyl group and carbolynyl group; arylvinyl groups such as stylyl group and naphthylvinyl group; acyl groups such as acetyl group and benzoyl group; and disubstituted amino groups having the above aromatic hydrocarbon groups or the aromatic heterocyclic groups as substituents, such as diphenylamino group, bis(dibenzofuranyl)amino group, bis(dibenzothienyl)amino group, dinaphthylamino group, phenyl-dibenzofuranylamino group, phenyl-dibenzothienylamino group and phenyl-naphthylamino group.

The above substituent may, further, have the substituent described above.

Further, though the substituents described above are, usually, present as independent groups, they may be singularly bonded or bonded together via a substituted or unsubstituted methylene group, an oxygen atom or a sulfur atom to form a ring.

In the invention, particularly preferred substituents are aromatic hydrocarbon group, sulfur-containing aromatic heterocyclic group, oxygen-containing aromatic heterocyclic group, N-phenylcarbazolyl group and disubstituted amino group, and more preferred substituents are phenyl group, biphenylyl group, naphthyl group, phenanthryl group, fluorenyl group, triphenylenyl group, dibenzofuranyl group, dibenzothienyl group, N-phenylcarbazolyl group and diphenylamino group.

In the invention, the above-mentioned $Ar^1$ and $Ar^2$ are the groups that may be the same or different from each other. Preferably, $Ar^1$ and $Ar^2$ are the groups that are different from each other.

In the invention, in particular, it is desired that $Ar^1$ and $Ar^2$ are different in regard to if they have a substituent. For instance, $Ar^1$ is, preferably, a phenyl without substituent while $Ar^2$ is a substituted phenyl group having the above substituent.

<$R^1$ to $R^{12}$>

In the above general formula (1), $R^1$ to $R^{12}$ are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, aryloxy groups or di-substituted amino groups having aromatic hydrocarbon groups or aromatic heterocyclic groups as substituents bonded to the nitrogen atom.

Among the groups denoted by $R^1$ to $R^{12}$, as the alkyl group having 1 to 6 carbon atoms, there can be exemplified methyl group, ethyl group, n-propyl group, isopropyl group, n-butyl group, isobutyl group, tert-butyl group, n-pentyl group,

isopentyl group, neopentyl group and n-hexyl group.

As the cycloalkyl group having 5 to 10 carbon atoms, there can be exemplified cyclopentyl group, cyclohexyl group, 1-adamantyl group and 2-adamantyl group.

Further, as the alkenyl group having 2 to 6 carbon atoms, there can be exemplified vinyl group, allyl group, isopropenyl group and 2-butenyl group.

As the alkyloxy group having 1 to 6 carbon atoms, there can be exemplified methyloxy group, ethyloxy group, n-propyloxy group, isopropyloxy group, n-butyloxy group, tert-butyloxy group, n-pentyloxy group and n-hexyloxy group.

As the cycloalkyloxy group having 5 to 10 carbon atoms, there can be exemplified cyclopentyloxy group, cyclohexyloxy group, cycloheptyloxy group, 1-adamantyloxy group and 2-adamantyloxy group.

As the above-mentioned aromatic hydrocarbon group and aromatic heterocyclic group, there can be exemplified by the same groups illustrated in the groups $Ar^1$ and $Ar^2$.

Further, as the above-mentioned aryloxy group, there can be exemplified phenyloxy group, biphenyloxy group, terphenylyloxy group, naphthyloxy group, anthryloxy group, phenanthryloxy group, fluorenyloxy group, indenyloxy group, pyrenyloxy group and perylenyloxy group.

As the aromatic hydrocarbon group and aromatic heterocyclic group which are contained by a disubstituted amino group as the substituent, there can be exemplified by the same groups illustrated in the groups $Ar^1$ and $Ar^2$.

The groups represented by $R^1$ to $R^{12}$ may have substituents so far as they do not hinder the limitation concerned to the carbon number, and these substituents may be the same as those possessed by the aromatic hydrocarbon group and the aromatic heterocyclic group represented by $Ar^1$ and $Ar^2$.

Further, $R^1$ to $R^{12}$ are, usually, present as independent groups. However, these groups may be singularly bonded or bonded to each other via a substituted or unsubstituted methylene group, an oxygen atom or a sulfur atom to form a ring.

In the invention, the above $R^1$ to $R^{12}$ are, preferably, hydrogen atoms, deuterium atoms, fluorine atoms, chlorine atoms, alkyl groups having 1 to 6 carbon atoms, aromatic hydrocarbon groups, sulfur-containing aromatic heterocyclic groups, oxygen-containing aromatic heterocyclic groups, N-phenylcarbazolyl groups or disubstituted amino groups and, more preferably, are hydrogen atoms, deuterium atoms, phenyl groups, biphenylyl groups, naphthyl groups, phenanthryl groups, fluorenyl groups, dibenzofuranyl groups, dibenzothienyl groups, N-phenylcarbazolyl groups or diphenylamino groups.

In the invention, it is more desired that $R^1$, $R^2$, $R^4$ to $R^9$, $R^{11}$ and $R^{12}$ are hydrogen atoms or deuterium atoms. That is, it is desired that, among $R^1$ to $R^{12}$, only $R^3$ and $R^{10}$ are the substituted groups which is substituted for a hydrogen atom (or a deuterium atom), and those other than $R^3$ and $R^{10}$ are hydrogen atoms.

<Concrete examples of the dicarbazole derivatives>

Described below are preferred concrete examples of the dicarbazole derivatives of the invention to which only, however, the invention is in no way limited.

Here, in the following concrete examples, the compound No. 1 is a missing number.

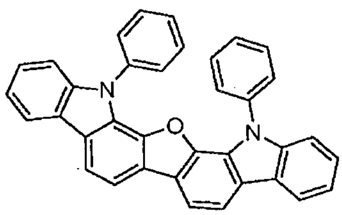

(Compound 2)

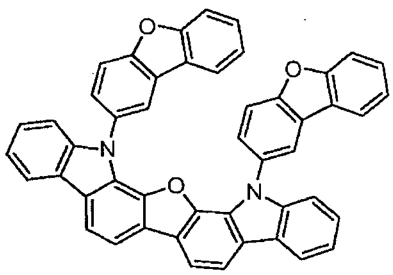

(Compound 3)

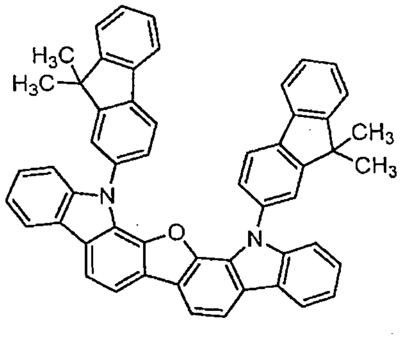

(Compound 4)

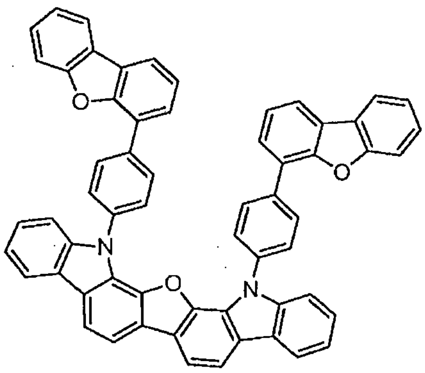

(Compound 5)

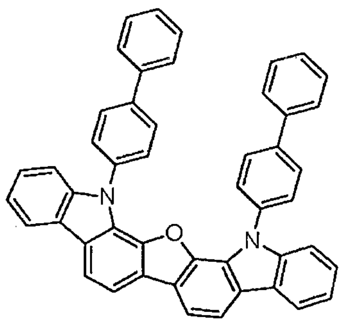

(Compound 6)

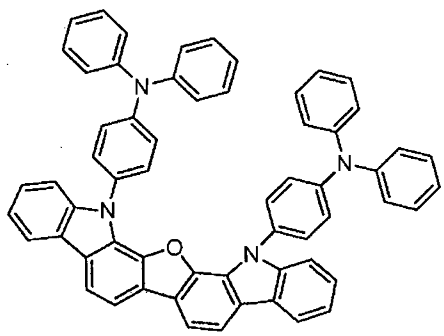

(Compound 7)

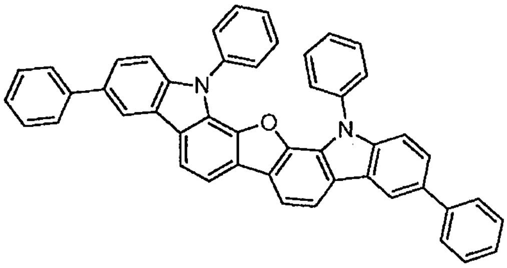

(Compound 8)

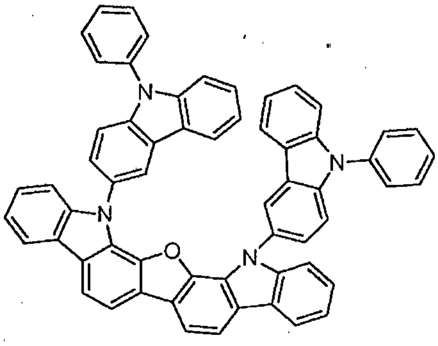

(Compound 9)

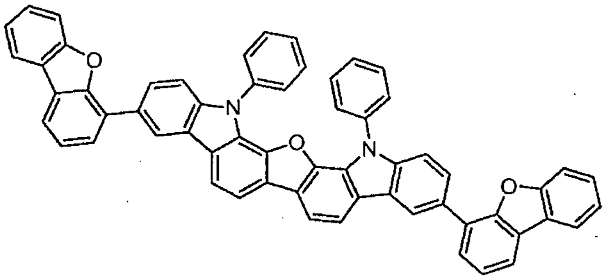

(Compound 10)

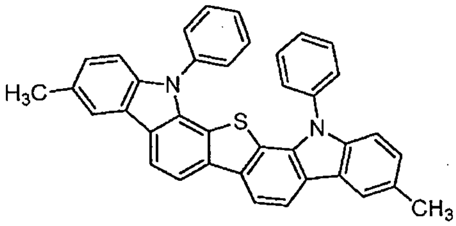

(Compound 11)

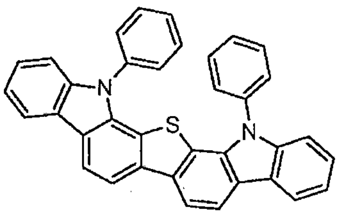

(Compound 12)

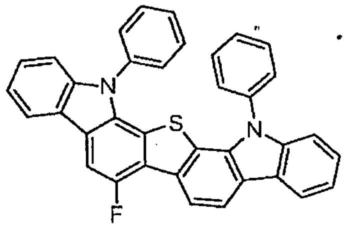

(Compound 13)

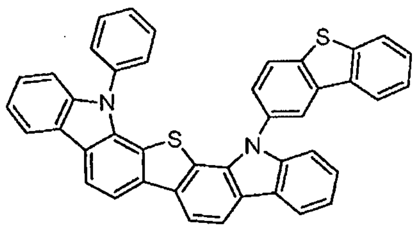

(Compound 14)

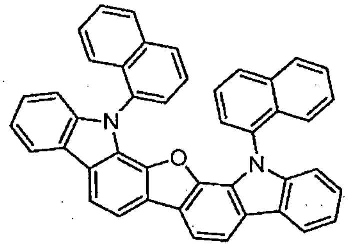

(Compound 15)

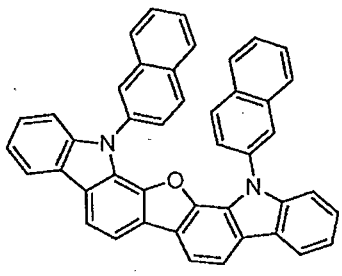

(Compound 16)

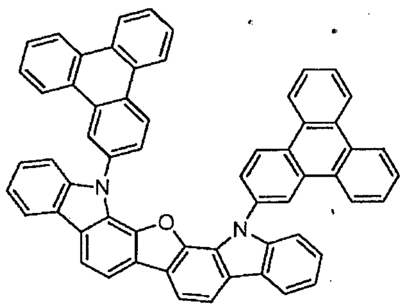

(Compound 17)

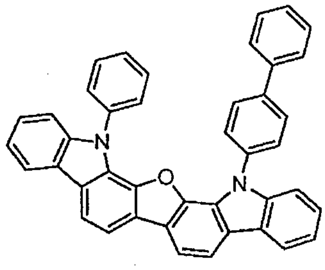

(Compound 18)

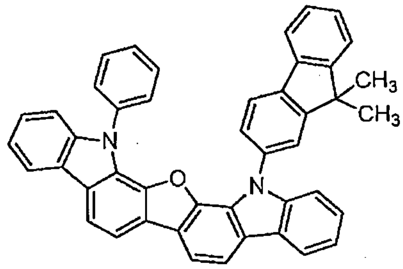

(Compound 19)

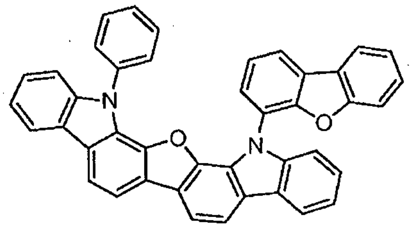

(Compound 20)

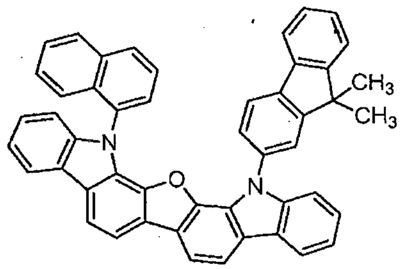

(Compound 21)

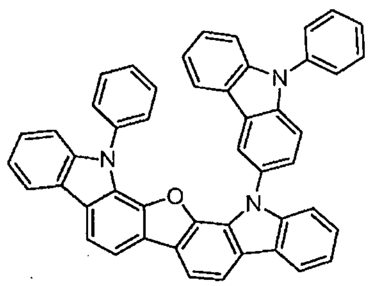

(Compound 22)

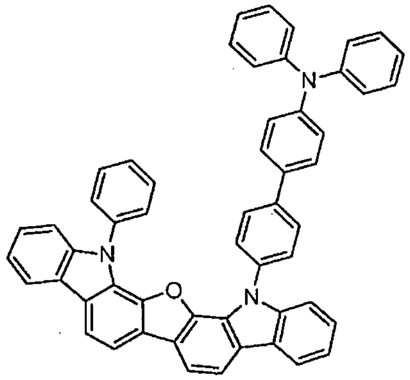

(Compound 23)

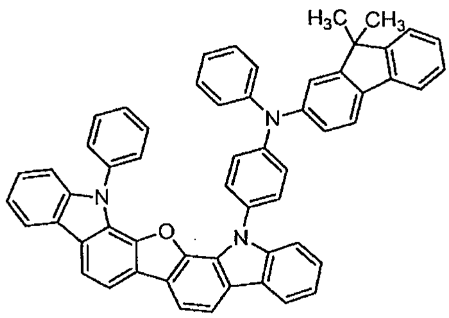

(Compound 24)

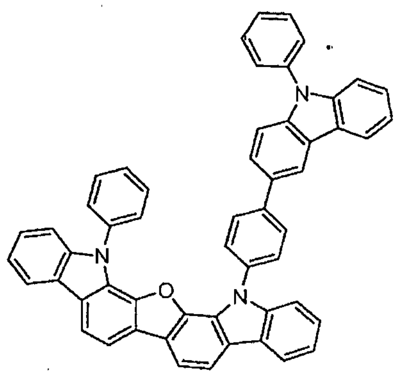

(Compound 25)

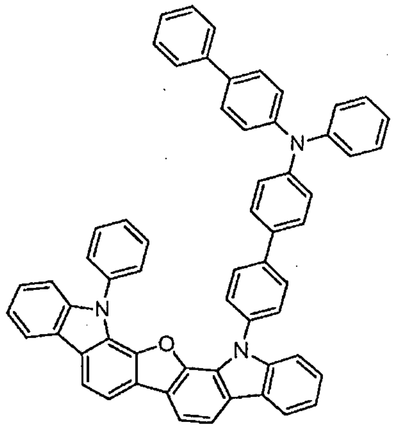

(Compound 26)

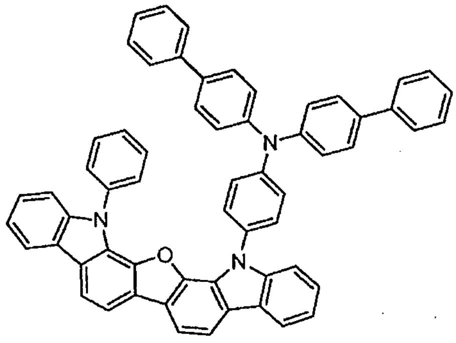

(Compound 27)

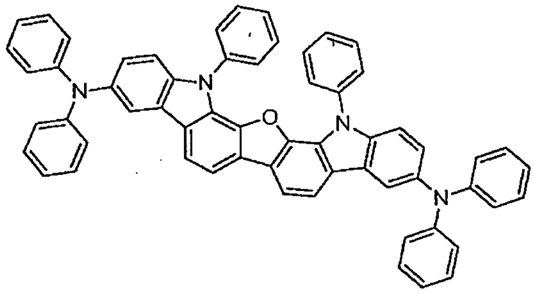

(Compound 28)

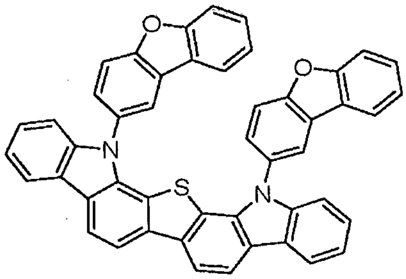

(Compound 29)

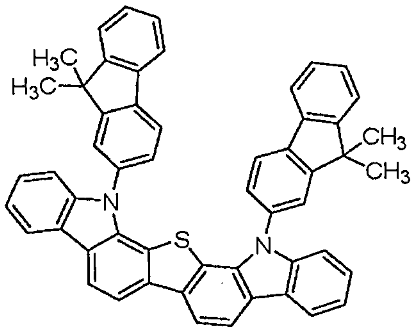

(Compound 30)

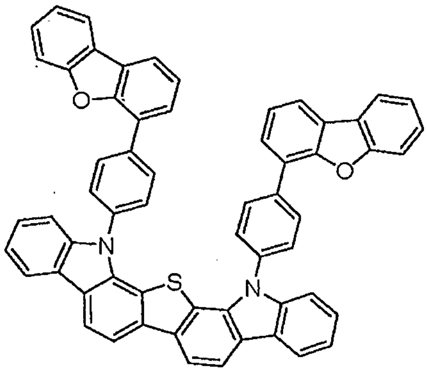

(Compound 31)

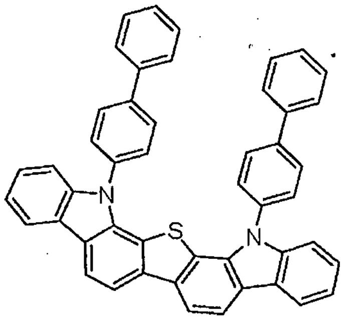

(Compound 32)

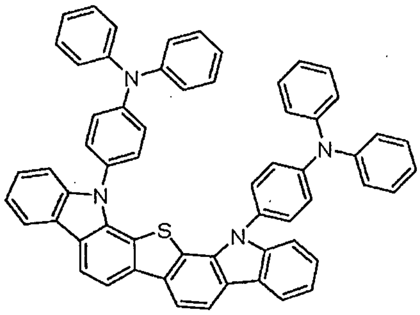

(Compound 33)

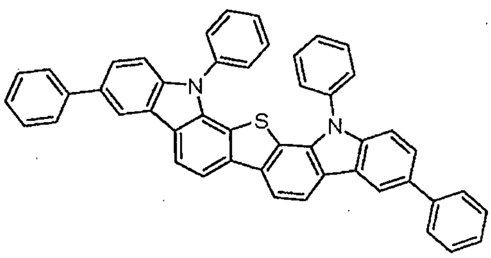

(Compound 34)

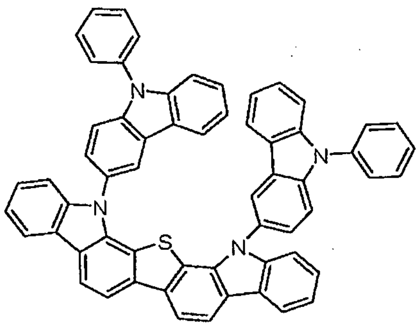

(Compound 35)

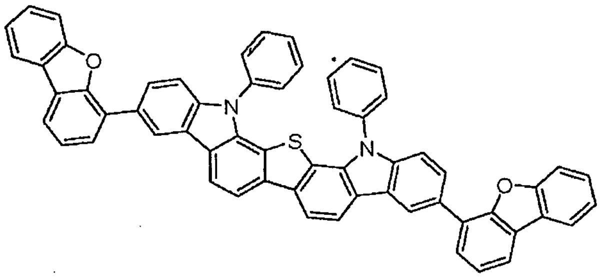

(Compound 36)

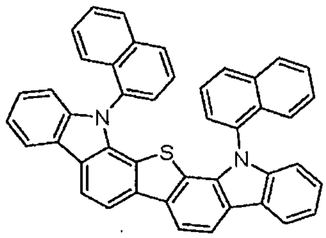

(Compound 37)

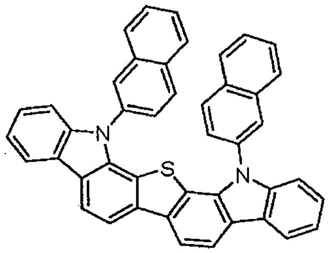

(Compound 38)

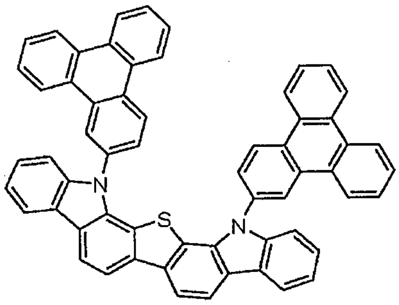

(Compound 39)

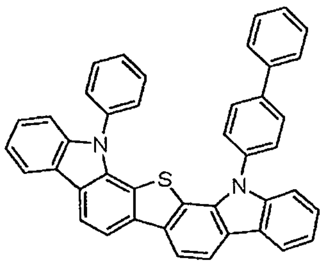

(Compound 40)

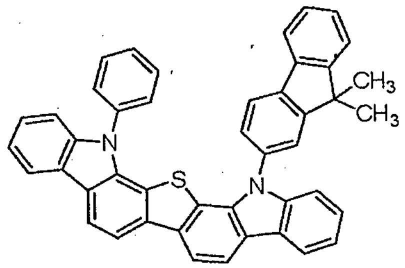

(Compound 41)

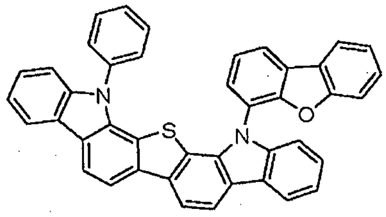

(Compound 42)

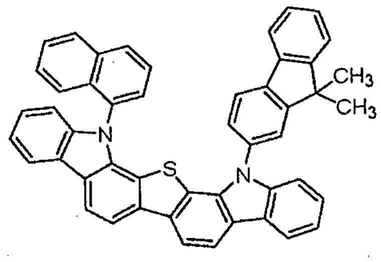

(Compound 43)

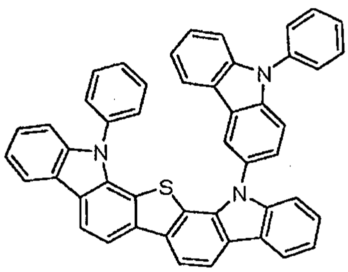

(Compound 44)

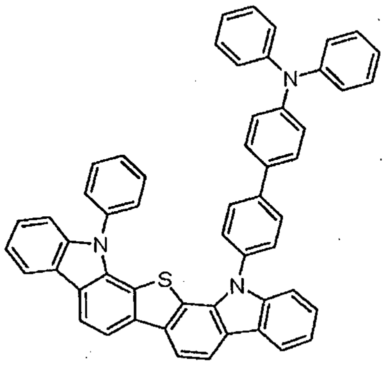

(Compound 45)

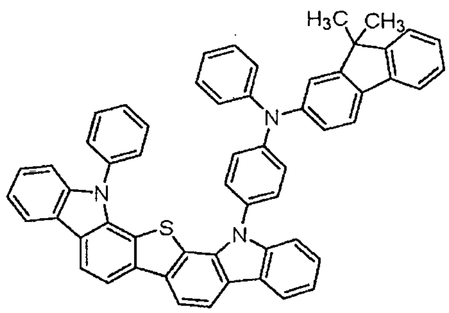

(Compound 46)

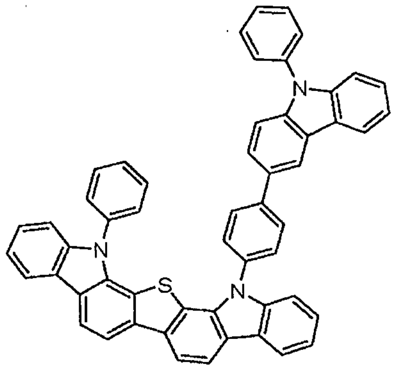

(Compound 47)

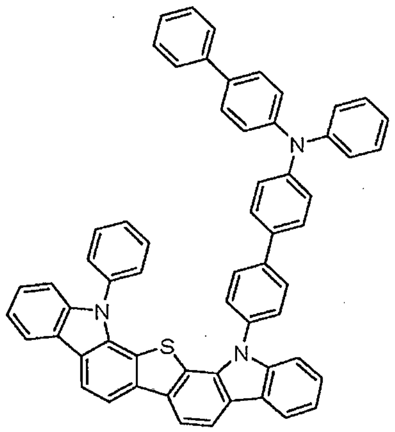

(Compound 48)

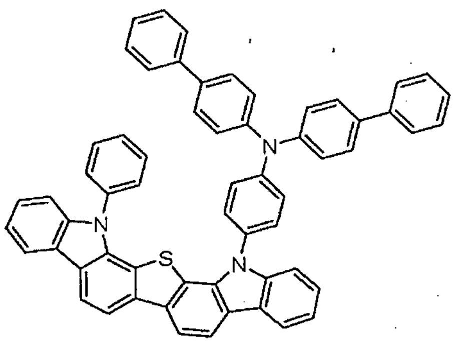

(Compound 49)

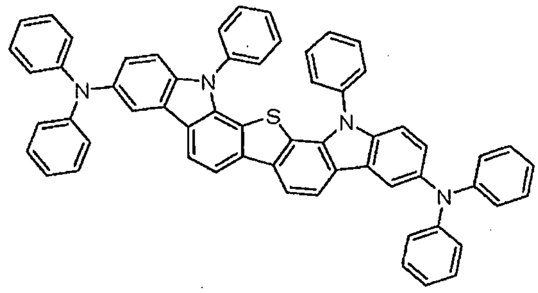

(Compound 50)

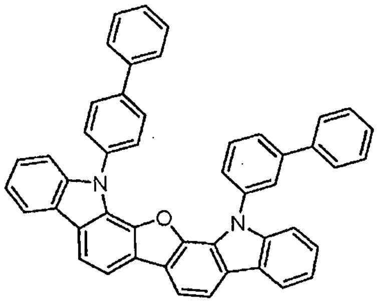

(Compound 51)

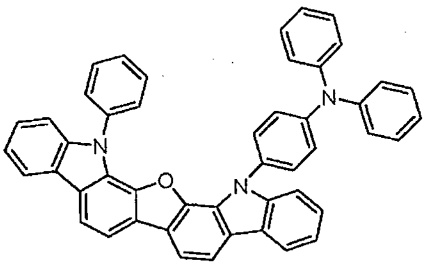

(Compound 52)

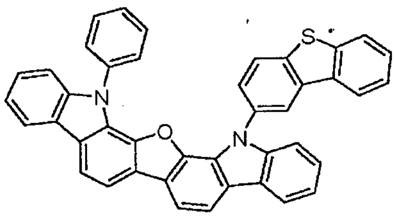

(Compound 53)

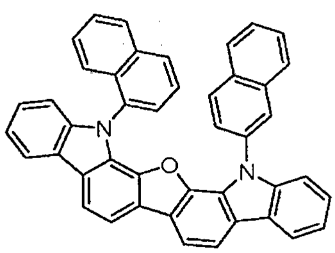

(Compound 54)

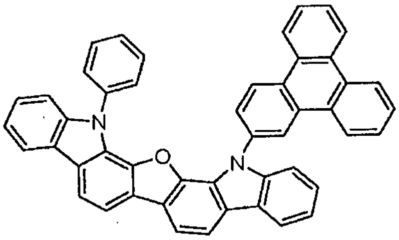

(Compound 55)

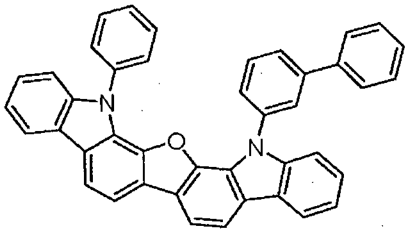

(Compound 56)

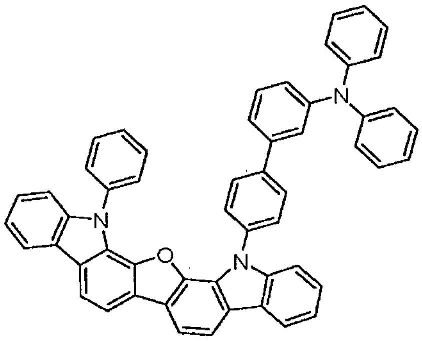

(Compound 57)

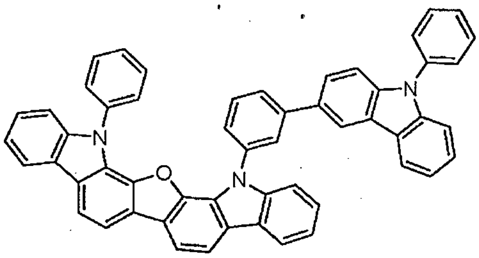

(Compound 58)

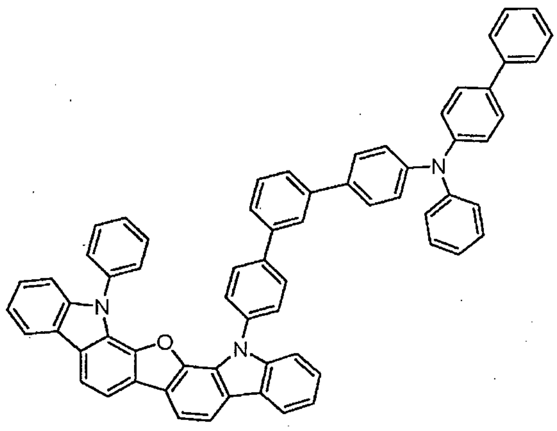

(Compound 59)

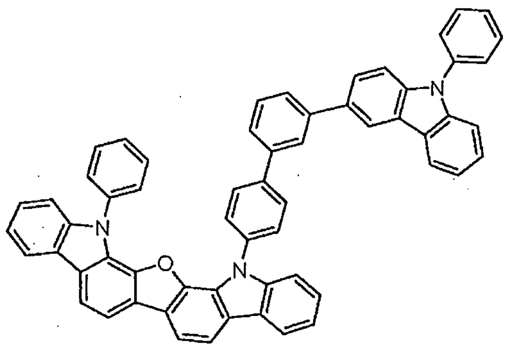

(Compound 60)

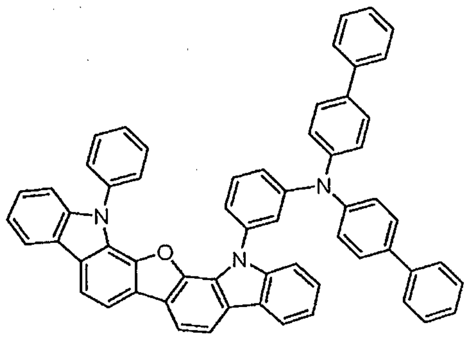

(Compound 61)

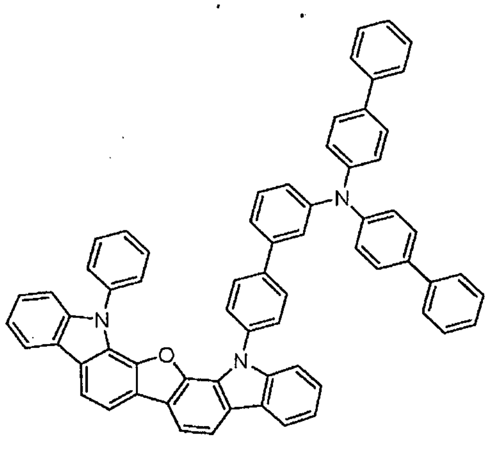

(Compound 62)

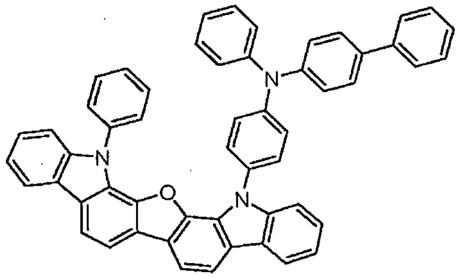

(Compound 63)

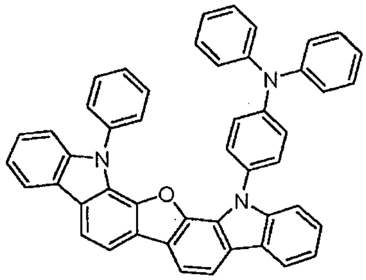

(Compound 64)

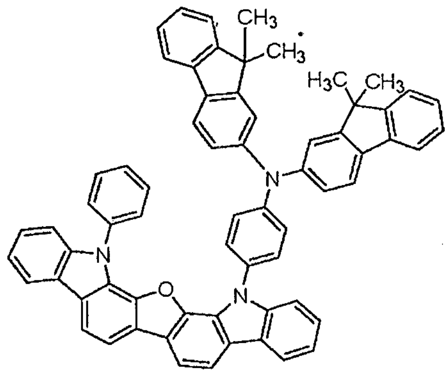

(Compound 65)

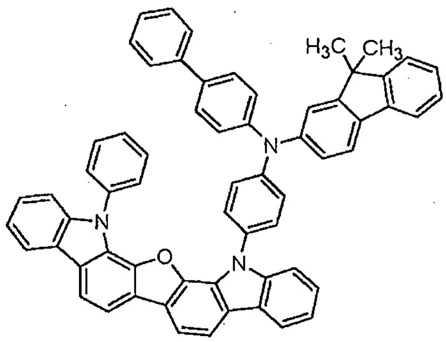

(Compound 66)

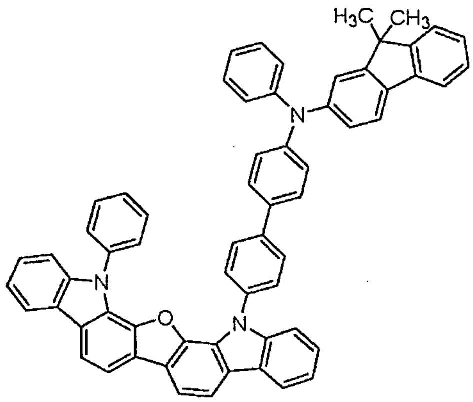

(Compound 67)

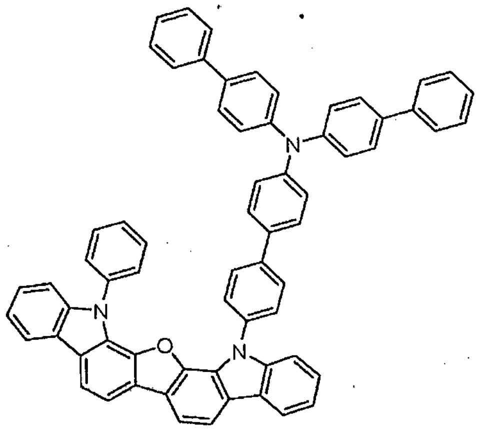

(Compound 68)

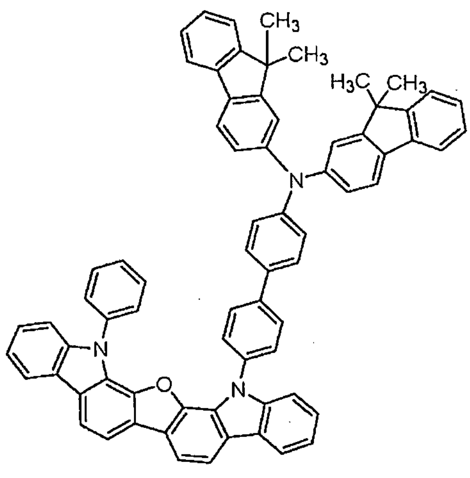

(Compound 69)

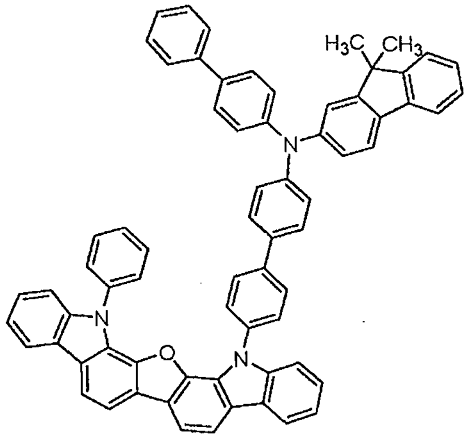

(Compound 70)

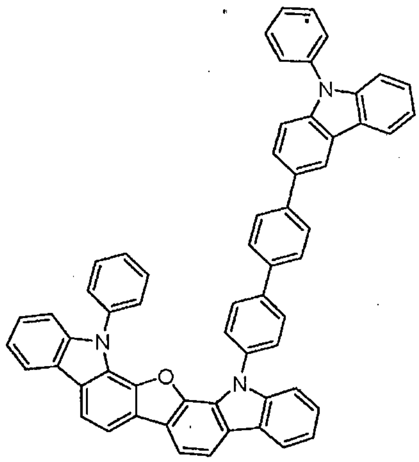

(Compound 71)

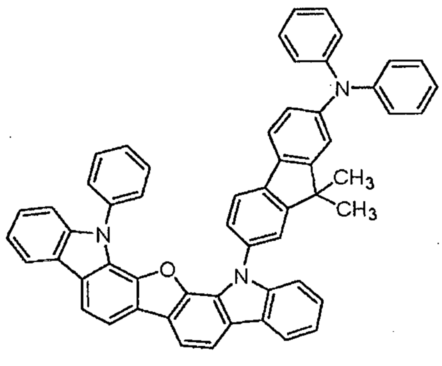

(Compound 72)

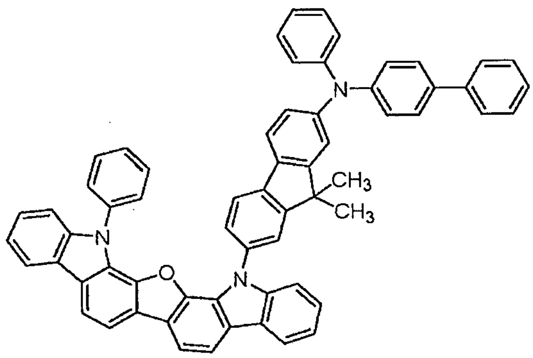

(Compound 73)

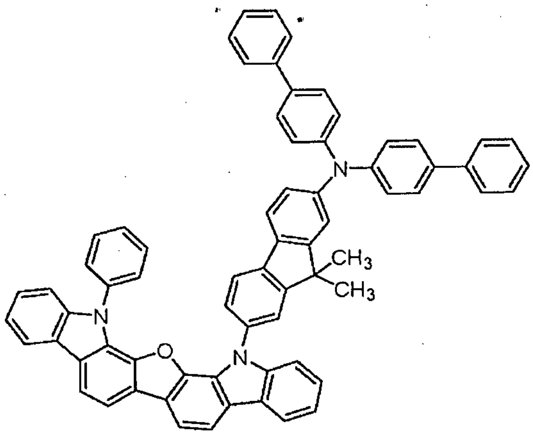

(Compound 74)

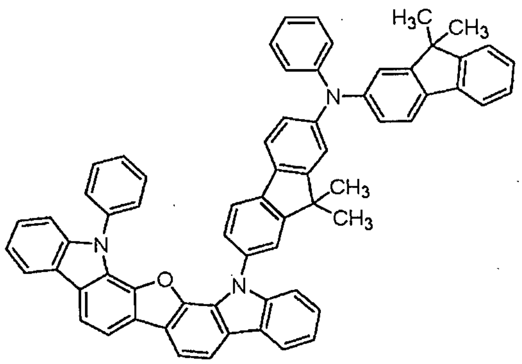

(Compound 75)

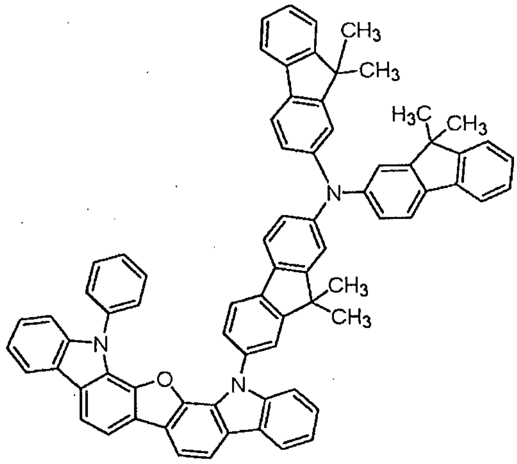

(Compound 76)

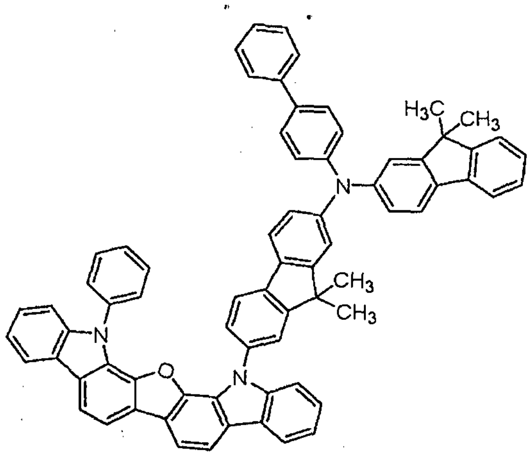

(Compound 77)

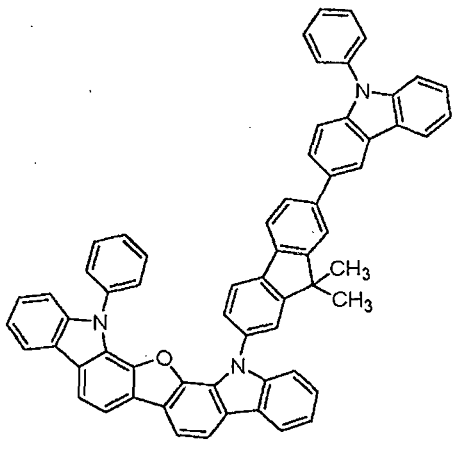

(Compound 78)

<Production of dicarbazole derivatives>

The dicarbazole derivatives of the present invention represented by the above-mentioned general formula (1) can be synthesized as described below.

For example, a 4,6-diiododibenzofurane is reacted with a 2-bromoaniline to synthesize an N,N-bis(2-bromophenyl)-dibenzofuran-4,6-diamine which is then subjected to the cyclization reaction to synthesize a furodicarbazole. Next, the furodicarbazole and an aryl halide are subjected to the condensation reaction based on, for example, the Buchwald-Hartwig reaction to synthesize a compound having a furodicarbazole ring structure of the invention (X is an oxygen atom in the general formula (1)).

Further, about an introduction of a substituent into the furodicarbazole ring structure, for example, a brominated furodicarbazole derivative may be synthesized by a bromination with an imide N-bromosuccinate. Here, by changing the reagent and the conditions for bromination, it is allowed to obtain bromo substituents having different positions of substitution. Further, by conducting the cross-coupling reaction such as Suzuki coupling using various boronic acids or boronic acid esters (e.g., see Synth. Commun. , 11, 513 (1981)), it is allowed to synthesize dicarbazole derivatives having the furodicarbazole ring structure of the present invention in which various kinds of substituents are introduced.

Further, instead of using the 4,6-diiododibenzofuran, a 4,6-diiododibenzothiophene may be reacted with the 2-bromoaniline, and the subsequent synthesizing reaction may be conducted in the same manner as described above to synthesize the dicarbazole derivatives of the invention having a thienodicarbazole ring structure.

The obtained compounds can be refined by the column chromatography, by the adsorptive refining using silica gel, active carbon or active clay, by the recrystallization using a solvent, or by the crystallization method. Further, the compounds can be identified by the NMR analysis.

The dicarbazole derivatives of the invention thus obtained have a high glass transition point (Tg), are capable of forming thin films having excellent heat resistance, remain in an amorphous state maintaining stability, and retain the state of thin films maintaining stability. Moreover, they have a high hole injection property, a high hole mobility and a high electron-blocking capability. For example, if a film of the compound of the invention is deposited in a thickness of 100 nm on an ITO substrate and if a work function is measured that serves as an index of hole-transporting property, then a very high value is exhibited.

Therefore, the dicarbazole derivatives of the invention are very useful as materials for forming organic layers (e.g., hole-transporting layer, electron-blocking layer, hole injection layer, luminous layer) of an organic EL device.

<Organic EL device>

Fig. 7 illustrates the structure of an organic EL device having organic layers formed by using the dicarbazole derivatives of the present invention.

Namely, on a glass substrate 1 (which may be a transparent substrate such as transparent plastic substrate), there are formed a transparent anode 2, a hole injection layer 3, a hole-transporting layer 4, a luminous layer 5, an electron-transporting layer 6, an electron injection layer 7 and a cathode 8.

Of course, the organic EL device for which the dicarbazole derivatives of the invention are used is not limited to the above-mentioned layer structure but may be the one that has an electron-blocking layer or the like formed between the hole-transporting layer 4 and the luminous layer 5, or the one that has a hole-blocking layer formed between the luminous layer 5 and the electron-transporting layer 6. Further, a simplified layer structure can also be employed omitting the electron injection layer 8 or the hole injection layer 3. For example, in the above-mentioned multilayer structure, some of the layers can be omitted. As an example, a simplified layer structure can be employed comprising the anode 2, hole-transporting layer 4, luminous layer 5, electron-transporting layer 6 and cathode 8 that are formed on the substrate 1.

The dicarbazole derivatives of the invention can be favorably used as materials for forming organic layers (e.g., hole injection layer 3, hole-transporting layer 4 and luminous layer 5, or electron-blocking layer suitably formed between the hole-transporting layer 4 and the luminous layer 5) that are formed between the anode 2 and the cathode 8.

In the above EL device, the transparent anode 2 can be formed by using an electrode material that is known per se., and is formed by depositing an electrode material having a large work function, such as ITO or gold on the base plate 1 (transparent base plate such as glass base plate).

Further, the hole injection layer 3 on the transparent anode 2 can be formed by using not only the dicarbazole derivatives of the invention but also the known materials such as those described below.

Porphyrin compound as represented by copper phthalocyanine;

Triphenylamine derivative of the star burst type;

Arylamine having a structure that is singularly bonded or bonded with a divalent group having no hetero atom (e. g. , triphenylamine trimer and tetramer);

Acceptor type heterocyclic compound such as hexacyanoazatriphenylene;

Application type high molecular material such as poly(3,4-ethylenedioxythiophene) (PEDOT), poly(styrene sulfonate) (PSS), etc.

The layers (thin films) can be formed by using the above materials not only by the vapor deposition method but also by such a known method as spin-coating method or ink-jet method. The layers mentioned below, too, can be formed by the vapor deposition method, spin-coating method, ink-jet method or the like.

The hole-transporting layer 4 on the hole injection layer 3, too, can be formed by using the known hole-transporting materials in addition to using the dicarbazole derivatives of the present invention. Described below are representative examples of the conventional hole-transporting materials. Benzidine derivatives such as:

N,N'-diphenyl-N,N'-di(m-tolyl)benzidine (hereinafter abbreviated as TPD);
N,N'-diphenyl-N,N'-di($\alpha$-naphthyl)benzidine (hereinafter abbreviated as NPD); and
N,N,N',N'-tetrabiphenylylbenzidine;

Amine type derivatives such as:

1,1-Bis[4-(di-4-tolylamino)phenyl] cyclohexane (hereinafter abbreviated as TAPC);
Various triphenylamine trimers and tetramers; and The above application type high molecular material that is also used for forming the hole injection layer.

The compounds for forming the hole-transporting layer may be used alone or in a mixture of two or more kinds. Further, a plurality of layers may be formed by using one kind or a plurality kinds of the above compounds, and a multilayer film of a lamination of such layers may be used as the hole-transporting layer.
Further, a layer may be so formed as to serve both as the hole injection layer 3 and the hole-transporting layer 4. Such a hole infection transporting layer can be formed by being coated with a high molecular material such as poly(3,4-ethylenedioxythiophene) (hereinafter abbreviated as PEDOT) or polystylene sulfonate (hereinafter abbreviated as PSS).
In the hole-transporting layer 4 (the same also holds for the hole injection layer 3), the material that is usually used for forming the layer may be P-doped with a trisbromophenylaminehexachloroantimony and be used. The hole-transporting layer 4 (or the hole injection layer 3) can also be formed by using a high molecular compound having a TPD basic skeleton.
Further, the electron-blocking layer (that can be provided between the hole-transporting layer 4 and the luminous layer 5), though not diagramed, can also be formed by using the dicarbazole derivatives of the invention as well as the known electron-blocking compounds having the action for blocking electrons, such as known carbazole derivatives and a compound that has a triphenylsilyl group and a triarylamine structure. Described below are concrete examples of the known carbazole derivatives and the compounds having the triarylamine structure.

<Known carbazole derivatives>

4,4',4''-Tri(N-carbazolyl)triphenylamine (hereinafter abbreviated as TCTA);
9,9-Bis[4-(carbazole-9-il)phenyl] fluorene;
1,3-Bis(carbazole-9-il) benzene (hereinafter abbreviated as mCP); and
2,2,-Bis(4-carbazole-9-ilphenyl)adamantane (hereinafter abbreviated as Ad-Cz).

<Compound having the triarylamine structure>

9-[4-(Carbazole-9-il)phenyl]-9-[4-(triphenylsilyl) phenyl]-9H-fluorene.
The electron-blocking layer is formed by using one kind or two or more kinds of the above electron-blocking materials. A plurality of layers may be formed by using one or a plurality of kinds of the electron-blocking materials, and a multilayer film of a laminate of these layers may be used as the electron-blocking layer.
The luminous layer 5 of the organic EL device can be formed by using the dicarbazole derivative of the invention as the luminous material. The luminous layer 5, however, can also be formed by using luminous materials, for example, a metal complex of a quinolynol derivative as represented by $Alq_3$, various metal complexes such as of zinc, beryllium and aluminum, anthracene derivatives, bisstyrylbenzene derivatives, pyrene derivatives, oxazole derivatives and polyparaphenylenevinylene derivatives.
It is also allowable to constitute the luminous layer 5 by using a host material and a dopant material.
As the host material in this case, there can be used thiazole derivatives, benzimidazole derivatives and polydialkylfluorene derivatives in addition to the above luminous materials.
As the dopant material, there can be used quinacridone, cumalin, rubrene, perylene and derivatives thereof, benzopyran derivatives, rhodamine derivatives and aminostyryl derivatives.
The luminous layer 5, too, can be formed in a single-layer structure or in a multilayer structure by laminating a plurality of layers by using one or two or more kinds of the luminous materials.
It is, further, allowable to form the luminous layer 5 by using a phosphorescent luminous material as the luminous material.
As the phosphorescent luminous material, there can be used a phosphorescent luminous body of a metal complex such as of iridium or platinum. For example, there can be used a green luminous phosphor such as $Ir(ppy)_3$, a blue luminous phosphor such as FIrpic or $FIr_6$, and a red luminous phosphor such as $Btp_2Ir(acac)$. These phosphorescent

luminous materials are used by being added to the hole injection transporting host material or to the electron-transporting host material.

As the hole injection transporting host material, there can be used 4,4'-di(N-carbazolyl)biphenyl (hereinafter abbreviated as CBP), or carbazole derivative such as TCTA or mCP in addition to using the dicarbazole derivatives of the present invention.

As the electron-transporting host material, there can be used p-bis (triphenylsilyl) benzene (hereinafter abbreviated as UGH2) or 2,2',2"-(1,3,5-phenylene)-tris(1-phenyl-1H-benzimidazole) (hereinafter abbreviated as TPBI).

To avoid the concentration quenching, the host material is desirably doped with the phosphorescent luminous material in an amount in a range of 1 to 30% by weight relative to the whole luminous layer relying on the vacuum coevaporation.

As the luminous material, further, it is also allowable to use a material that emits retarded fluorescence, such as CDCB derivatives like PIC-TRZ, CC2TA, PXZ-TRZ or 4CzlPN (see Appl. Phys. Let., 98, 083302 (2011)).

The hole-blocking layer (not shown) can be suitably formed between the luminous layer 5 and the electron-transporting layer 6 by using a known compound having the hole-blocking action.

As the known compounds having the hole-blocking action, there can be exemplified the following compounds.
Phenanthrolene derivatives such as bathocuproin (hereinafter abbreviated as BCP) and the like;
Metal complexes of quinolinol derivatives such as aluminum (III) bis(2-methyl-8-quinolinato)-4-phenylphenolate (hereinafter abbreviated as BAlq) and the like;
Various rare earth complexes;
Triazole derivatives;
Triazine derivatives; and
Oxadiazole derivatives.

These materials can also be used for forming the electron-transporting layer 6 that will be described below. Moreover, the hole-blocking layer and the electron-transporting layer 6 can be formed as one layer.

The hole-blocking layer, too, can be formed in the structure of a single layer or of a laminate of a multiplicity of layers, the layers being formed by using one kind or two or more kinds of the above-mentioned compounds having hole-blocking action.

The electron-transporting layer 6 can be formed by using electron-transporting compounds that have been known per se. such as metal complexes of quinolinol derivatives like $Alq_3$, BAlq, as well as various metal complexes, triazole derivatives, triazine derivatives, oxadiazole derivatives, thiadiazole derivatives, carbodiimide derivatives, quinoxaline derivatives, phenanthroline derivatives and silole derivatives.

The electron-transporting layer 6, too, can be formed in the structure of a single layer or of a laminate of a multiplicity of layers, the layers being formed by using one kind or two or more kinds of the above-mentioned electron-transporting compounds.

The electron injection layer 7, too, can be formed by using known compounds, i. e. , by using alkali metal salts such as lithium fluoride and cesium fluoride, alkaline earth metal salts such as magnesium fluoride, and metal oxides such as aluminum oxide.

As the electron injection layer 7 or the electron-transporting layer 6, further, it is also allowable to use the material that has usually been used for forming these layers but is, further, N-doped with a metal such as cesium or the like.

As the cathode 8 of the organic EL device, there can be used an electrode material having a low work function, such as aluminum, or an electrode material of an alloy having a lower work function, such as magnesium-silver alloy, magnesium-indium alloy or aluminum-magnesium alloy.

The organic EL device forming at least one of the organic layers (e.g., hole injection layer 3, hole-transporting layer 4, luminous layer 5 or electron-blocking layer) by using the dicarbazole derivative of the present invention, features a high luminous efficiency, a high power efficiency, a low practical driving voltage, a low luminance start voltage and very excellent durability.

## EXAMPLES

Though the invention will now be more concretely described by way of Examples, it should be noted that the invention is in no way limited to these Examples only.

<Example 1>

Synthesis of a 5,7-dihydro-5,7-bis(9,9-dimethylfluorene-2-il)-furo[2,3-a:5,4-a'] dicarbazole.

(Synthesis of the compound 4)

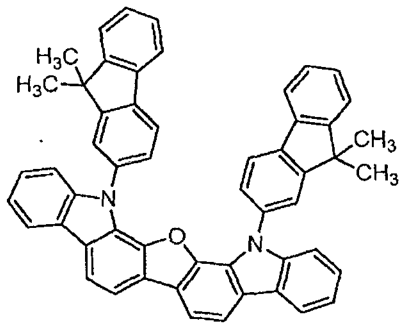

(Compound 4)

Into a reaction vessel purged with nitrogen,

| | |
|---|---|
| N,N,N',N'-tetramethylethylenediamine | 10.4 g and |
| THF | 40 ml |

were put and cooled and to which a hexane solution of n-butyl lithium (1.6 mols/L) was added dropwise while maintaining the temperature of the solution at not higher than 0°C.

Further, the solution was stirred for 30 minutes at 0°C and for another 30 minutes at room temperature.

Thereafter, 25 ml of THF and 5.0 g of dibenzofuran were added thereto, and the mixture was heated and stirred at 60°C for 2 hours. Next, a 1,2-diiodoethane was added thereto while being cooled at -60°C or lower and, thereafter, the mixture was stirred overnight at room temperature. After water and dichloromethane were added, the organic layer was picked up by the liquid separation operation. The organic layer was dehydrated with the anhydrous magnesium sulfate and was, thereafter, concentrated under reduced pressure to obtain a crude product.

Heptane was added to the crude product, the mixture thereof was stirred, and the precipitated product was picked up by filtration to obtain 2.7 g of a pale yellow powder of a 4,6-diiododibenzofuran (yield, 22%).

| | |
|---|---|
| 4,6-Diiododibenzofuran obtained above | 2.7 g, |
| 2-bromoaniline | 2.2 g, |
| tert-butoxysodium | 1.5 g and |
| toluene | 27 ml |

were put into the reaction vessel purged with nitrogen, and the mixture thereof was aerated with a nitrogen gas for one hour.

Thereafter, to the reaction vessel,

| | |
|---|---|
| tris(dibenzilideneacetone) dipalladium (O) | 0.2 g and |
| diphenylphosphinoferrocene | 0.3 g |

were added. Then, the mixture was heated and stirred at 100°C for 4 hours. The mixture was cooled down to room temperature, the insoluble matter was removed by filtration, and the filtrate was concentrated under reduced pressure to obtain a crude product.

The crude product was refined by the column chromatography (carrier: silica gel, eluent: toluene/n-hexane) to obtain 2.4 g of a pale brown powder of an $N^4$,$N^6$-bis(2-bromophenyl)-dibenzofuran-4,6-diamine (yield, 73%).

To the reaction vessel purged with nitrogen, there were added;

| | |
|---|---|
| $N^4$,$N^6$-bis(2-bromophenyl)-dibenzofuran-4,6-diamine obtained above | 2.4 g, |

(continued)

| | |
|---|---|
| potassium acetate | 1.9 g, |
| DMF | 12 ml and |
| water | 2 ml. |

The above mixture was aerated with the nitrogen gas for one hour and to which was, further, added 0.2 g of a tetrakis(triphenylphosphine) palladium followed by heating and stirring at 72°C for 12 hours. The mixture was cooled down to room temperature and to which 30 ml of water and 30 ml of toluene were added. The solid material was picked up by filtration to obtain 0.5 g of a pale brown powder of a 5,7-dihydro-furo[2,3-a:5,4-a'] dicarbazole (yield, 30%).

| | |
|---|---|
| 5,7-Dihydro-furo[2,3-a:5,4-a']dicarbazole obtained above, | 7.5 g, |
| 2-iodo-9,9-dimethylfluorene | 17.3 g, |
| copper iodide | 0.2 g, |
| tripotassium phosphate | 13.8 g, |
| 1,2-cyclohexanediamine | 7.4 g and |
| 1,4-dioxane | 60 ml |

were put into the reaction vessel purged with nitrogen, and were stirred at 95°C for 45 hours. The mixture thereof was cooled down to room temperature and to which 60 ml of water and 60 ml of toluene were added, followed by the extraction operation with toluene to pick up the organic layer. The organic layer was dehydrated with the anhydrous magnesium sulfate and was concentrated under reduced pressure to obtain a crude product.

The crude product was refined by the column chromatography (carrier: silica gel, eluent: toluene/n-heptane) to obtain 13.8 g of a white powder of the 5,7-dihydro-5,7-bis(9,9-dimethylfluorene-2-il)- furo[2,3-a:5,4-a']dicarbazole (compound 4) (yield, 87%).

The obtained white powder was identified for its structure by the NMR. Fig. 1 shows the results of the $^1$H-NMR measurement.

The following 26 signals of hydrogen were detected by the $^1$H-NMR ($CDCl_3$).

$$\delta\,(\mathrm{ppm}) = 8.21\ (4H)$$
$$8.03\ (2H)$$
$$7.30 - 7.50\ (16H)$$
$$7.18\ (4H)$$

<Example 2>

Synthesis of a 5,7-dihydro-5,7-bis{4-(dibenzofurane-4-il) phenyl}-furo[2,3-a:5,4-a']dicarbazole.

(Synthesis of the compound 5)

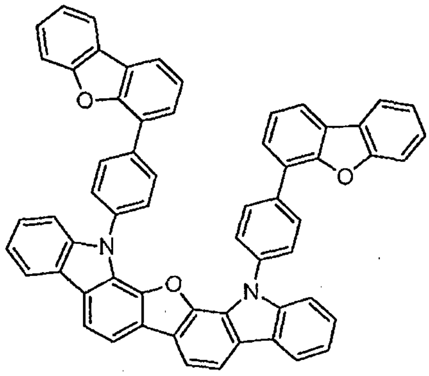

(Compound 5)

To the reaction vessel purged with nitrogen, there were added;

| | |
|---|---|
| 5,7-dihydro-furo[2,3-a:5,4-a']dicarbazole synthesized in Example 1 | 6.0 g, |
| 4-(4-bromophenyl)dibenzofuran | 12.3 g, |
| tert-butoxysodium | 5.0 g and |
| toluene | 60 ml |

and the mixture thereof was aerated with the nitrogen gas for one hour.

Thereafter, to the reaction vessel,

| | |
|---|---|
| tris(dibenzilideneacetone) dipalladium (O) | 0.6 g and |
| toluene solution containing 50% (w/v) of tri-tert -butylphosphine | 0.8 g |

were added. Then, the mixture was heated and stirred at 95°C for 18 hours. The mixture was cooled down to room temperature, water was added thereto, the extraction operation was conducted with toluene, and an organic layer was picked up. The organic layer was dehydrated with the anhydrous magnesium sulfate and was concentrated under reduced pressure to obtain a crude product.

The crude product was refined by the column chromatography (carrier: silica gel, eluent: toluene/n-hexane) to obtain 2.7 g of a white powder of the 5,7-dihydro-5,7-bis{4-(dibenzofuran-4-il)phenyl}-furo[2,3-a:5,4-a']dicarbazole (compound 5) (yield, 18%).

The obtained white powder was identified for its structure by the NMR. Fig. 2 shows the results of the $^1$H-NMR measurement.

The following 34 signals of hydrogen were detected by the $^1$H-NMR ($CDCl_3$).

$$\delta\,(ppm) = 8.23\ (4H)$$
$$8.04\ (2H)$$
$$7.77 - 7.90\ (6H)$$
$$7.32 - 7.57\ (20H)$$
$$7.08\ (2H)$$

<Example 3>

Synthesis of a 5,7-dihydro-5,7-bis(biphenyl-4-il)-furo[2,3-a:5,4-a']dicarbazole.

(Synthesis of the compound 6)

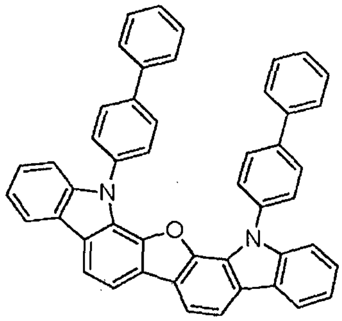

(Compound 6)

To the reaction vessel purged with nitrogen, there were added;

| | |
|---|---|
| 5,7-dihydro-furo[2,3-a:5,4-a']dicarbazole synthesized in Example 1 | 7.4 g, |
| 4-iodobiphenyl | 13.2 g, |
| tert-butoxysodium | 6.2 g and |
| toluene | 70 ml |

and the mixture thereof was aerated with the nitrogen gas for one hour.

Thereafter, to the reaction vessel,

| | |
|---|---|
| tris(dibenzilideneacetone) dipalladium (O) | 0.8 g and |
| toluene solution containing 50% (w/v) of tri-tert-butylphosphine | 1.0 g |

were added. Then, the mixture was heated and stirred at 95°C for 29 hours. The mixture was cooled down to room temperature, water was added thereto, the extraction operation was conducted with toluene, and an organic layer was picked up. The organic layer was dehydrated with the anhydrous magnesium sulfate and was concentrated under reduced pressure to obtain a crude product.

The crude product was refined by the column chromatography (carrier: silica gel, eluent: toluene/n-hexane) to obtain 6.0 g of a white powder of the 5,7-dihydro-5,7-bis(biphenyl-4-il)-furo [2,3-a:5,4-a']dicarbazole (compound 6) (yield, 433%).

The obtained white powder was identified for its structure by the NMR. Fig. 3 shows the results of the $^1$H-NMR measurement.

The following 30 signals of hydrogen were detected by the $^1$H-NMR ($CDCl_3$).

$$\delta\,(\mathrm{ppm}) = 8.18 - 8.22\ (4H)$$
$$8.01\ (2H)$$
$$7.30 - 7.50\ (24H)$$

<Example 4>

Synthesis of a 5,7-dihydro-5-{4-(9-phenylcarbazole-3-il) phenyl}-7-phenyl-furo[2,3-a:5,4-a']dicarbazole.

(Synthesis of the compound 25)

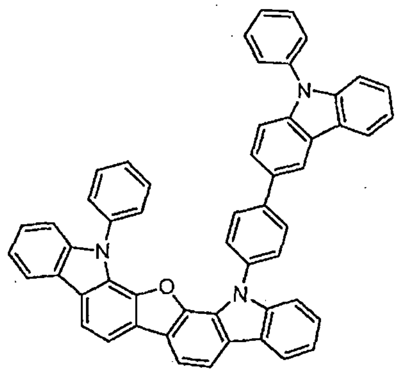

(Compound 25)

To the reaction vessel purged with nitrogen, there were added;

| | |
|---|---|
| 5,7-dihydro-furo[2,3-a:5,4-a']dicarbazole synthesized in Example 1 | 7.0 g, |
| 3-(4-bromophenyl)-9-phenylcarbazole | 10.5 g, |
| cesium carbonate | 9.9 g and |
| xylene | 70 ml |

and the mixture thereof was aerated with the nitrogen gas for one hour.

Thereafter, to the reaction vessel,

| | |
|---|---|
| tris(dibenzilideneacetone) dipalladium (O) | 0.9 g and |
| toluene solution containing 50% (w/v) of tri-tert-butylphosphine | 0.8 g |

were added. Then, the mixture was heated and stirred at 110°C for 42 hours. The mixture was cooled down to room temperature, and 70 ml of water was added thereto.

The precipitating solid matter was picked up by filtration and was washed with 70 ml of a mixed solvent of methanol/water (5/1 v/v). 200 Milliliters of 1,2-dichlorobenzene was added thereto and the mixture was heated so the solid matter was dissolved therein.

The insoluble matter was removed by filtration from the solution, the solution was left to cool, 100 ml of heptane was added thereto, and the precipitating crude product was picked up by filtration to obtain 8.2 g of a grey powder of the 5,7-dihydro-5-{4-(9-phenylcarbazole-3-il) phenyl}-furo [2,3-a:5,4-a']dicarbazole (yield, 61%).

To the reaction vessel purged with nitrogen, there were added;

| | |
|---|---|
| 5,7-dihydro-5-{4-(9-phenylcarbazol-3-il)phenyl}-furo [2,3-a:5,4-a']dicarbazole obtained above | 8.2 g, |
| iodobenzene | 3.8 g, |
| copper iodide | 0.1 g, |
| tripotassium phosphate | 3.9 g, |
| 1,2-cyclohexanediamine | 2.1 g and |
| 1,4-dioxane | 70 ml |

and the mixture thereof was heated and stirred at 95°C for 20 hours. The mixture was cooled down to room temperature, 70 ml of water and 70 ml of toluene were added thereto, the extraction operation was conducted with toluene, and an organic layer was picked up.

The organic layer was dehydrated with the anhydrous magnesium sulfate and was concentrated under reduced pressure to obtain a crude product.

The crude product was refined by the column chromatography (carrier: silica gel, eluent: toluene/n-heptane) to obtain 7.0 g of a white powder of the 5,7-dihydro-5-{4-(9-phenylcarbazole-3-il)phenyl}-7-phenyl-furo[2,3-a:5,4-a']dicarbazole (compound 25) (yield, 77%).

The obtained white powder was identified for its structure by the NMR. Fig. 4 shows the results of the $^1$H-NMR measurement.

The following 33 signals of hydrogen were detected by the $^1H$-NMR ($CDCl_3$).

$\delta$ (ppm) = 8.58 (1H)
8.34 (1H)
8.18 - 8.25 (4H)
8.00 (2H)
7.90 (1H)
7.62 - 7.77 (7H)
7.49 - 7.60 (6H)
7.30 - 7.50 (8H)
7.09 (2H)
6.88 (1H)

<Example 5>

Synthesis of a 5,7-dihydro-5-[4'-{(biphenyl-4-il)-phenylamino}biphenyl-4-il]-7-phenyl-furo[2,3-a:5,4-a'] dicarbazole.

(Synthesis of the compound 26)

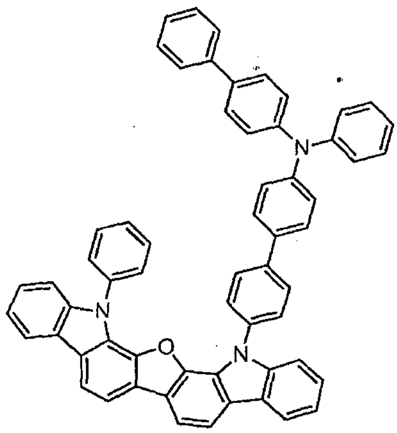

(Compound 26)

To the reaction vessel purged with nitrogen, there were added;

| | |
|---|---|
| 5,7-dihydro-furo[2,3-a:5,4-a']dicarbazole synthesized in Example 1 | 7.0 g, |
| N-(biphenyl-4-il)-N-phenyl-(4'-bromobiphenyl-4-il) amine | 15.5 g, |
| cesium carbonate | 19.8 g and |
| xylene | 70 ml |

and the mixture thereof was aerated with the nitrogen gas for one hour.

Thereafter, to the reaction vessel,

| | |
|---|---|
| tris(dibenzilideneacetone) dipalladium (O) | 0.9 g and |
| toluene solution containing 50% (w/v) of tri-tert-butylphosphine | 0.8 g |

were added. Then, the mixture was heated and stirred at 110°C for 15 hours. The mixture was cooled down to room temperature, and 70 ml of water was added thereto.

The precipitating solidmatter was picked up by filtration and was washed with 70 ml of a mixed solvent of methanol/water (5/1 v/v). 200 Milliliters of 1,2-dichlorobenzene was added thereto and the mixture was heated so the solid matter was dissolved therein.

The insoluble matter was removed by filtration from the solution, the solution was left to cool, 100 ml of methanol was added thereto, and the precipitating crude product was picked up by filtration to obtain 7.8 g of a grey powder of the 5,7-dihydro-5-[4'-{(biphenyl-4-il)- phenylamino} biphenyl-4-il]-furo[2,3-a:5,4-a']dicarbazole (yield, 52%).

To the reaction vessel purged with nitrogen, there were added;

| | |
|---|---|
| 5,7-dihydro-5-[4'-{(biphenyl-4-il)-phenylamino} biphenyl-4-il]-furo[2,3-a:5,4-a']dicarbazole obtained above | 7.6 g, |
| iodobenzene | 3.2 g, |
| copper iodide | 0.1 g, |
| tripotassium phosphate | 3.4 g, |
| 1,2-cyclohexanediamine | 1.8 g and |
| 1,4-dioxane | 60 ml |

and the mixture thereof was heated and stirred at 95°C for 22 hours. The mixture was cooled down to room temperature, 70 ml of water and 70 ml of toluene were added thereto, the extraction operation was conducted with toluene, and an organic layer was picked up. The organic layer was dehydrated with the anhydrous magnesium sulfate and was concentrated under reduced pressure to obtain a crude product.

The crude product was refined by the column chromatography (carrier: silica gel, eluent: toluene/n-heptane) to obtain 7.6 g of a white powder of the 5, 7-dihydro-5- [4'-{(biphenyl-4-il)-phenylamino}biphenyl-4-il]-7-phenyl-furo[2,3-a:5,4-a'] dicarbazole (compound 26) (yield, 88%).

The obtained white powder was identified for its structure by the NMR. Fig. 5 shows the results of the $^1$H-NMR measurement.

The following 39 signals of hydrogen were detected by the $^1$H-NMR ($CDCl_3$).

$$\begin{aligned}\delta\,(\mathrm{ppm}) = {} & 8.15 - 8.25\ (4H)\\ & 7.99\ (2H)\\ & 7.72\ (2H)\\ & 7.54 - 7.70\ (6H)\\ & 7.28 - 7.51\ (17H)\\ & 7.18 - 7.24\ (6H)\\ & 6.94\ (2H)\end{aligned}$$

<Example 6>

Synthesis of a 5,7-dihydro-5-[4-{bis(biphenyl-4-il)amino} phenyl]-7-phenyl-furo[2,3-a:5,4-a']dicarbazole.

(Synthesis of the compound 27)

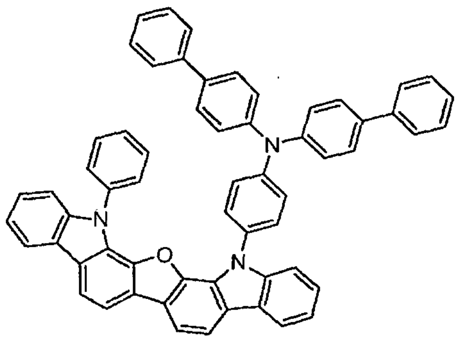

(Compound 27)

To the reaction vessel purged with nitrogen, there were added;

| | |
|---|---|
| 5,7-dihydro-furo[2,3-a:5,4-a']dicarbazole synthesized in Example 1 | 6.0 g, |
| (4-bromophenyl)-bis(biphenyl-4-il)amine | 8.3 g, |
| cesium carbonate | 8.5 g and |
| xylene | 60 ml |

and the mixture thereof was aerated with the nitrogen gas for one hour.

Thereafter, to the reaction vessel,

| | |
|---|---|
| tris(dibenzilideneacetone) dipalladium (O) | 0.5 g and |
| toluene solution containing 50% (w/v) of tri-tert-butylphosphine | 0.4 g |

were added. Then, the mixture was heated and stirred at 110°C for 40 hours. The mixture was cooled down to room temperature, and 60 ml of water was added thereto. The precipitating solid matter was picked up by filtration and was washed with 60 ml of a mixed solvent of methanol/water (5/1 v/v). 200 Milliliters of 1, 2-dichlorobenzene was added thereto and was heated so the solid matter was dissolved therein.

The insoluble matter was removed by filtration from the solution, the solution was left to cool, 100 ml of n-heptane was added thereto, and the precipitating crude product was picked up by filtration to obtain 8.5 g of a grey powder of the 5,7-dihydro-5-[4-{bis(biphenyl-4-il) amino}phenyl]-furo[2,3-a:5,4-a']dicarbazole (yield, 66%).

To the reaction vessel purged with nitrogen, there were added;

| | |
|---|---|
| 5,7-dihydro-5-[4-{bis(biphenyl-4-il)amino}phenyl]-furo[2,3-a:5,4-a']dicarbazole obtained above | 8.5 g, |
| iodobenzene | 3.5 g, |
| copper iodide | 0.1 g, |
| tripotassium phosphate | 3.7 g, |
| 1,2-cyclohexanediamine | 2.0 g and |
| 1,4-dioxane | 70 ml |

and the mixture thereof was heated and stirred at 95°C for 19 hours. The mixture was cooled down to room temperature, and 70 ml of water and 70 ml of n-heptane were added thereto.

The precipitating solid matter was picked up by filtering, washed with 70 ml of a mixed solvent of methanol/water (5/1 v/v) . Thereafter, 100 ml of 1, 2-dichlorobenzene was added thereto and was heated so the solid matter was dissolved therein.

The insoluble matter was removed by filtration from the solution, the solution was left to cool, 100 ml of n-heptane was added thereto, and the precipitating crude product was picked up by filtration. The crude product was reflux-washed with 100 ml of methanol to obtain 7.0 g of a pale brown powder of the 5,7-dihydro-5-[4-{bis(biphenyl-4-il) amino}phenyl]-7-phenyl-furo[2,3-a:5,4-a']dicarbazole (compound 27) (yield, 75%).

The obtained pale brown powder was identified for its structure by the NMR. Fig. 6 shows the results of the $^{1}$H-NMR measurement.

The following 39 signals of hydrogen were detected by the $^{1}$H-NMR ($CDCl_3$).

$$\delta\,(ppm) = 8.16 - 8.22\ (4H)$$
$$7.99\ (2H)$$
$$7.64 - 7.70\ (8H)$$
$$7.30 - 7.58\ (21H)$$
$$7.27\ (2H)$$
$$7.03\ (2H)$$

<Example 7>

The compounds obtained in the above Examples 1 to 6 were measured for their glass transition points by using a highly sensitive differential scanning calorimeter (DSC3100SA manufactured by Bruker AXS K.K.). The results were as follows:

| | Glass transition points |
|---|---|
| Compound of Example 1 | 159°C |
| Compound of Example 2 | 162°C |
| Compound of Example 3 | 128°C |
| Compound of Example 4 | 155°C |
| Compound of Example 5 | 145°C |
| Compound of Example 6 | 149°C |

The results tell that the compounds of the present invention have glass transition points which are not lower than 100°C and, specifically, not lower than 120°C indicating that the thin films formed by using the compounds of the invention maintain stability.

<Example 8>

By using the compounds of the invention obtained in the above Examples 1 to 6, films were vapor-deposited in a thickness of 100 nm on an ITO substrate and were measured for their work functions by using an apparatus for measuring ionization potentials (Model PYS-202 manufactured by Sumitomo Heavy Industries, Ltd.). The results were as follows:

| | Work functions |
|---|---|
| Compound of Example 1 | 5.89 eV |
| Compound of Example 2 | 5.89 eV |
| Compound of Example 3 | 5.90 eV |
| Compound of Example 4 | 5.94 eV |
| Compound of Example 5 | 5.82 eV |
| Compound of Example 6 | 5.76 eV |

As described above, the compounds of the present invention have energy levels superior to a work function of 5.54 eV possessed by general hole-transporting materials such as NPD, TPD and the like and, therefore, have favorable hole-transporting capabilities.

<Example 9>

An organic EL device of a structure shown in Fig. 7 was fabricated by using the compound (compound 4) obtained in Example 1.

Concretely, the glass substrate 1 having the ITO film formed thereon in a thickness of 150 nm was washed with an organic solvent and was, thereafter, washed for its surface by the oxygen plasma treatment. Thereafter, the glass substrate with the ITO electrode was placed in a vacuum evaporation machine, and the pressure therein was reduced down to 0.001 Pa or lower.

Next, as the hole injection layer 3, a compound (HIM-1) of the following structural formula was formed in a

thickness of 20 nm so as to cover the transparent anode 2.

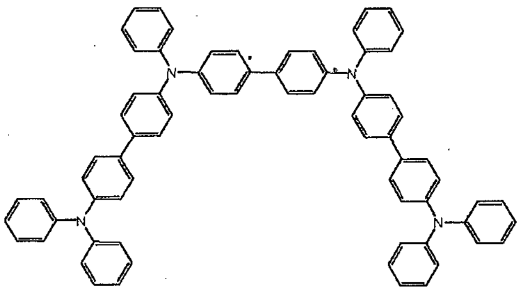

(HIM-1)

On the hole injection layer 3, the hole-transporting layer 4 was formed in a thickness of 40 nm by using the compound (compound 4) obtained in Example 1.

On the thus formed hole-transporting layer 4, the luminous layer 5 was formed in a thickness of 30 nm by two-way-depositing a compound (EMD-1) of the following structural formula and a compound (EMH-1) of the following structural formula at a deposition rate of EMD-1:EMH-1 = 5:95.

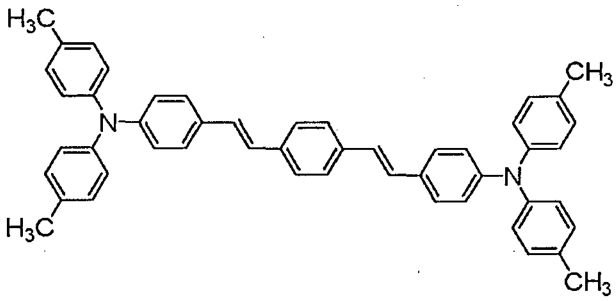

(EMD-1)

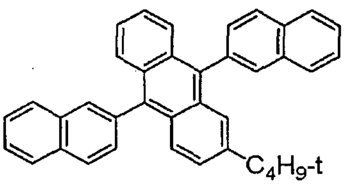

(EMH-1)

On the luminous layer 5, the electron-transporting layer 6 was formed by using the $Alq_3$ in a thickness of 30 nm. On the electron-transporting layer 6 was formed the electron injection layer 7 by using the lithium fluoride in a thickness of 0.5 nm. Finally, aluminum was deposited in a thickness of 150 nm to form the cathode 8.

The organic EL device fabricated above was measured for its properties in the atmosphere at normal temperature. Table 1 collectively shows the measured results of luminous characteristics of when a DC voltage is applied thereto.

<Example 10>

An organic EL device was fabricated in the same manner as in Example 9 but forming the hole-transporting layer 4 in a thickness of 40 nm by using the compound of Example 2 (compound 5) instead of using the compound of Example 1 (compound 4), and was evaluated to obtain the results as shown in Table 1.

<Example 11>

An organic EL device was fabricated in the same manner as in Example 9 but forming the hole-transporting layer 4 in a thickness of 40 nm by using the compound of Example 3 (compound 6) instead of using the compound of Example 1 (compound 4), and was evaluated to obtain the results as shown in Table 1.

<Example 12>

An organic EL device was fabricated in the same manner as in Example 9 but forming the hole-transporting

layer 4 in a thickness of 40 nm by using the compound of Example 4 (compound 25) instead of using the compound of Example 1 (compound 4), and was evaluated to obtain the results as shown in Table 1.

<Example 13>

An organic EL device was fabricated in the same manner as in Example 9 but forming the hole-transporting layer 4 in a thickness of 40 nm by using the compound of Example 5 (compound 26) instead of using the compound of Example 1 (compound 4), and was evaluated to obtain the results as shown in Table 1.

<Example 14>

An organic EL device was fabricated in the same manner as in Example 9 but forming the hole-transporting layer 4 in a thickness of 40 nm by using the compound of Example 6 (compound 27) instead of using the compound of Example 1 (compound 4), and was evaluated to obtain the results as shown in Table 1.

<Comparative Example 1>

For comparison, an organic EL device was fabricated in the same manner as in Example 9 but forming the hole-transporting layer 4 in a thickness of 40 nm by using a compound (HTM-1) of the following structural formula instead of using the compound of Example 1 (compound 4), and was evaluated to obtain the results as shown in Table 1.

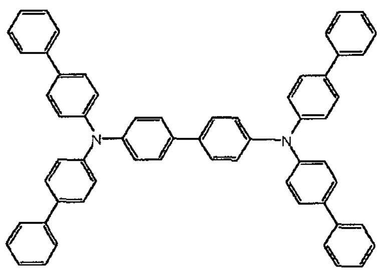

(HTM-1)

Table 1

| | Compound | Voltage [V] (@10 mA/cm$^2$) | Brightness [cd/m$^2$] (@10 mA/cm$^2$) | Luminous efficiency [cd/A] (@10 mA/cm$^2$) | Power efficiency [lm/W] (@10 mA/cm$^2$) |
|---|---|---|---|---|---|
| Example 9 | compound 4 | 4.72 | 906 | 9.06 | 6.03 |
| Example 10 | compound 5 | 4.75 | 911 | 9.11 | 6.03 |
| Example 11 | compound 6 | 4.70 | 924 | 9.24 | 6.18 |
| Example 12 | compound 25 | 4.85 | 1087 | 10.88 | 7.05 |
| Example 13 | compound 26 | 4.90 | 1100 | 11.00 | 7.13 |
| Example 14 | compound 27 | 4.92 | 1055 | 10.55 | 6.73 |
| Comp. Example 1 | HTM-1 | 5.17 | 902 | 9.03 | 5.49 |

When an electric current is flown at a current density of 10 mA/cm$^2$ as shown in Table 1, the organic EL devices using the compounds of Examples 1 to 6 of the present invention all drive at voltages of as low as 4.70 to 4. 92 V in comparison with 5.17 V at which the organic EL device using the HTM-1 drives.

As for the luminous efficiency, the organic EL devices using the compounds of Examples 1 to 6 of the present invention exhibit values of 9.06 to 11.00 cd/A which are great improvements over 9.03 cd/A of the organic EL device

that uses the HTM-1.

As for the power efficiency, too, the organic EL devices using the compounds of Examples 1 to 6 of the present invention exhibit values of 6.03 to 7.13 lm/W which are great improvements over 5.49 lm/W of the organic EL device that uses the HTM-1.

As is obvious from the above results, it is learned that the organic EL devices using the dicarbazole derivatives of the invention that have the furodicarbazole ring structure or the thienodicarbazole ring structure are capable of achieving higher power efficiencies and lower practical driving voltages than those of the organic EL device that uses the known compound.

Industrial Applicability:

The dicarbazole derivatives of the present invention have high hole-transporting power, excellent electron-blocking power, excellent amorphousness and remains stable in their thin film state, and can be favorably used as compounds for fabricating the organic EL devices. Upon fabricating the organic EL devices by using the above compounds, it is allowed to attain a high luminous efficiency and a high power efficiency, to lower the practical driving voltage and to improve the durability. Their use can, therefore, be expanded to, for example, domestic appliances and illumination equipment.

Description of Symbols:

1 glass substrate
2 transparent anode
3 hole injection layer
4 hole-transporting layer
5 luminous layer
6 electron-transporting layer
7 electron injection layer
8 cathode

## Claims

**1.** Dicarbazole derivatives represented by the following general formula (1),

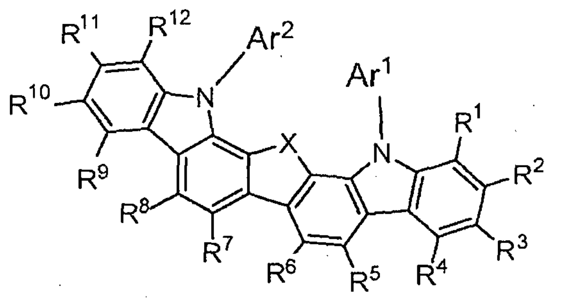

(1)

wherein,

X is an oxygen atom or a sulfur atom,
$Ar^1$ and $Ar^2$ are aromatic hydrocarbon groups or aromatic heterocyclic groups, and
$R^1$ to $R^{12}$ are hydrogen atoms, deuterium atoms, fluorine atoms, chlorine atoms, cyano groups, nitro groups, alkyl groups having 1 to 6 carbon atoms, cycloalkyl groups having 5 to 10 carbon atoms, alkenyl groups having 2 to 6 carbon atoms, alkyloxy groups having 1 to 6 carbon atoms, cycloalkyloxy groups having 5 to 10 carbon atoms, aromatic hydrocarbon groups, aromatic heterocyclic groups, aryloxy groups or disubstituted amino groups having aromatic hydrocarbon groups or aromatic heterocyclic groups as substituents bonded to the nitrogen atom, $R^1$ to $R^{12}$ may be singularly bonded or bonded to each other via a methylene group, an oxygen atom or a sulfur atom to form a ring.

**2.** Dicarbazole derivatives according to claim 1, wherein in the above general formula (1), X is an oxygen atom and the dicarbazole derivatives have a furodicarbazole ring structure.

**3.** Dicarbazole derivatives according to claim 1, wherein in the above general formula (1), X is a sulfur atom and the dicarbazole derivatives have a thienodicarbazole ring structure.

**4.** Dicarbazole derivatives according to claim 1, wherein in the above general formula (1), $R^1$, $R^2$, $R^9$ to $R^9$, $R^{11}$ and $R^{12}$ are hydrogen atoms or deuterium atoms.

**5.** Dicarbazole derivatives according to claim 4, wherein in the above general formula (1), $R^1$, $R^2$, $R^4$ to $R^9$, $R^{11}$ and $R^{12}$ are hydrogen atoms.

**6.** Dicarbazole derivatives according to claim 1, wherein in the above general formula (1), $Ar^1$ is an unsubstituted phenyl group.

**7.** Dicarbazole derivatives according to claim 6, wherein $Ar^1$ is an unsubstituted phenyl group, and $Ar^2$ is a group different from $Ar^1$.

**8.** Dicarbazole derivatives according to claim 7, wherein $Ar^2$ is a phenyl group having a substituent.

**9.** An organic electroluminescent device comprising a pair of electrodes and at least one organic layer sandwiched therebetween, wherein the dicarbazole derivative described in claim 1 is used as a material for constituting at least one organic layer.

**10.** The organic electroluminescent device according to claim 9, wherein the organic layer formed by using the dicarbazole derivative is a hole-transporting layer.

**11.** The organic electroluminescent device according to claim 9, wherein the organic layer formed by using the dicarbazole derivative is an electron-blocking layer.

**12.** The organic electroluminescent device according to claim 9, wherein the organic layer formed by using the dicarbazole derivative is a hole injection layer.

**13.** The organic electroluminescent device according to claim 9, wherein the organic layer formed by using the dicarbazole derivative is a luminous layer.

Fig. 1

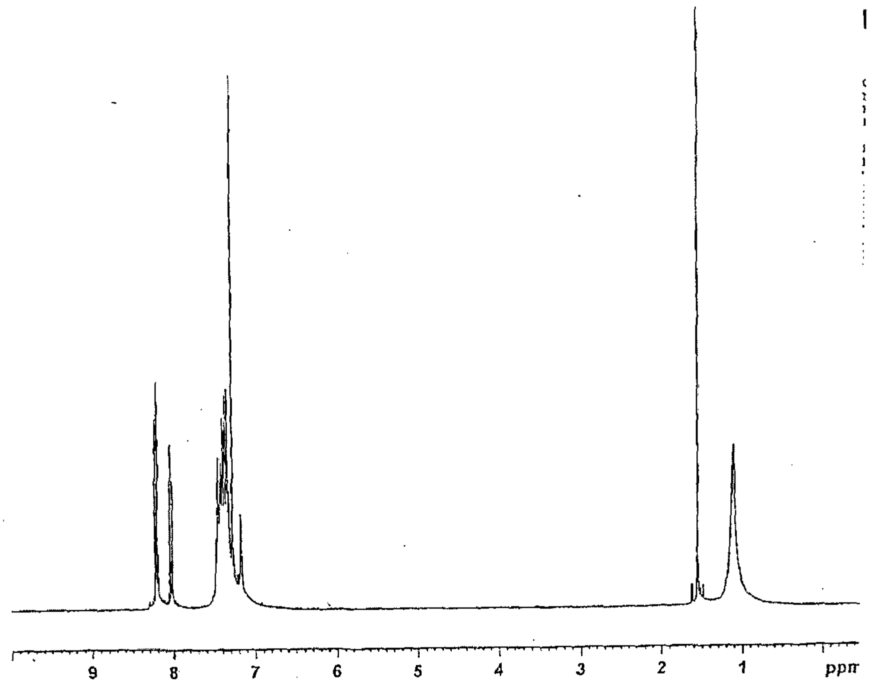


Fig. 2

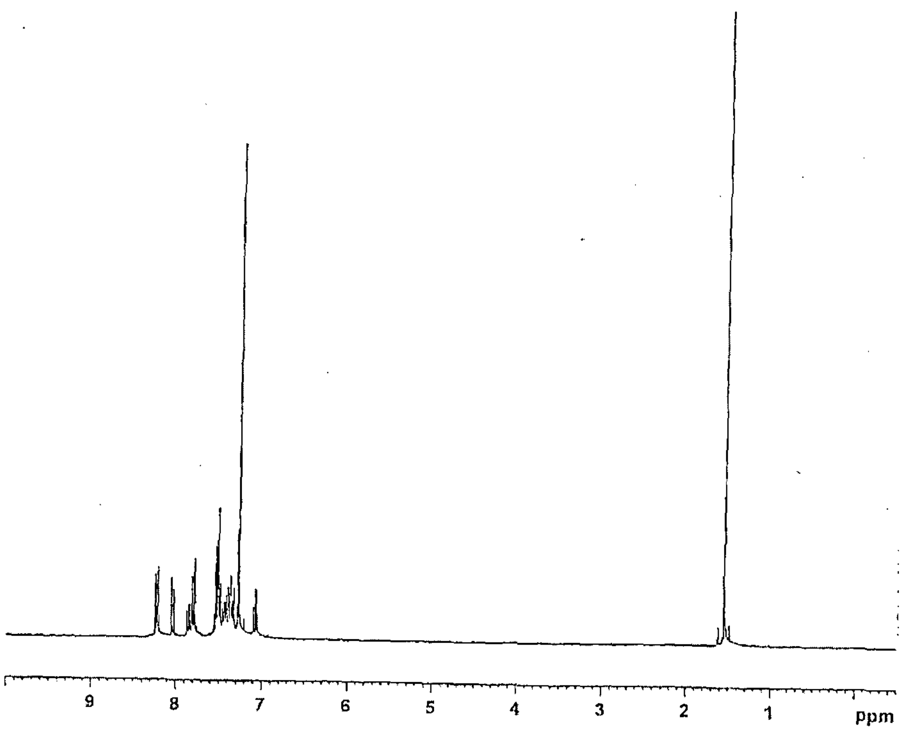

Fig. 3

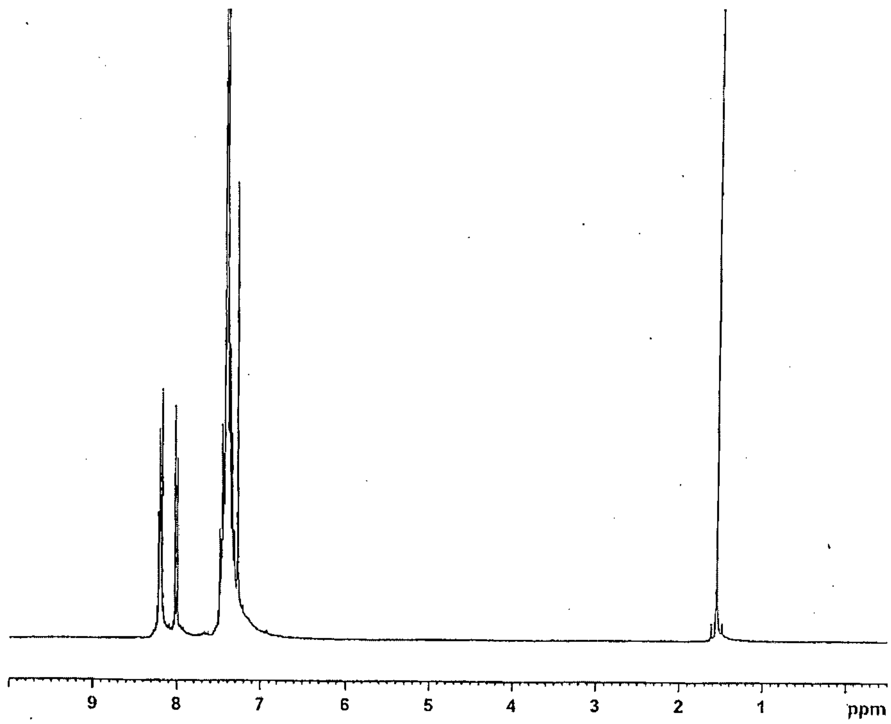


Fig. 4

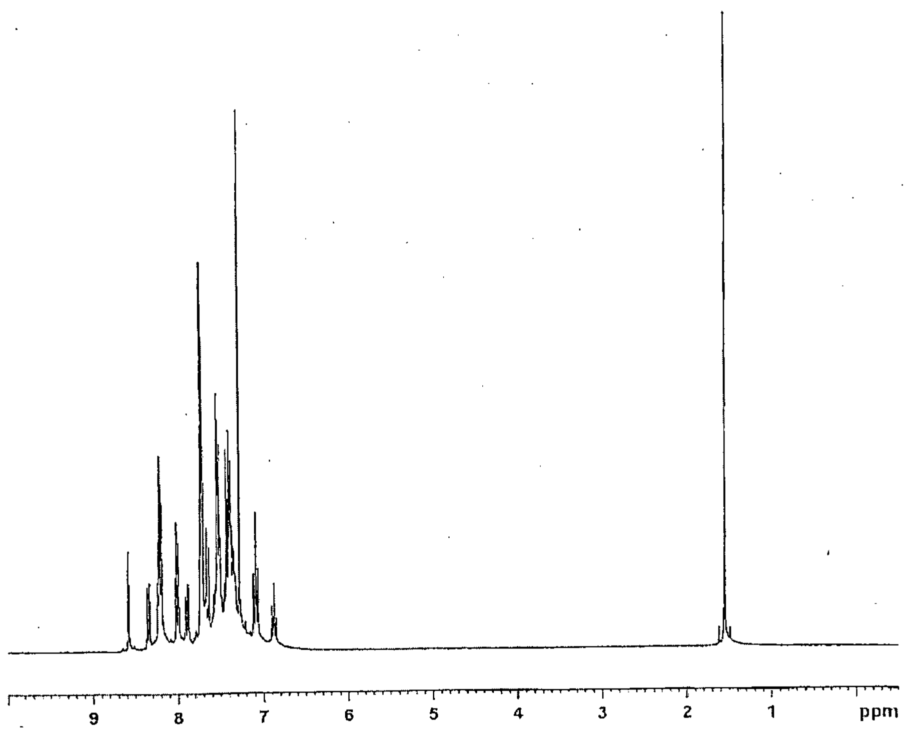

Fig. 5

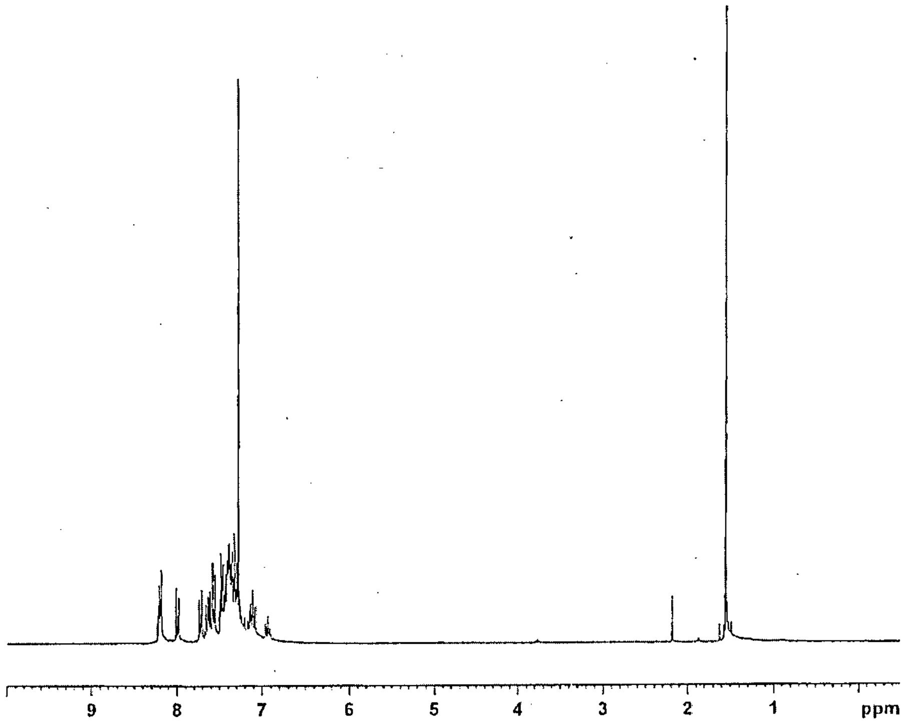


Fig. 6

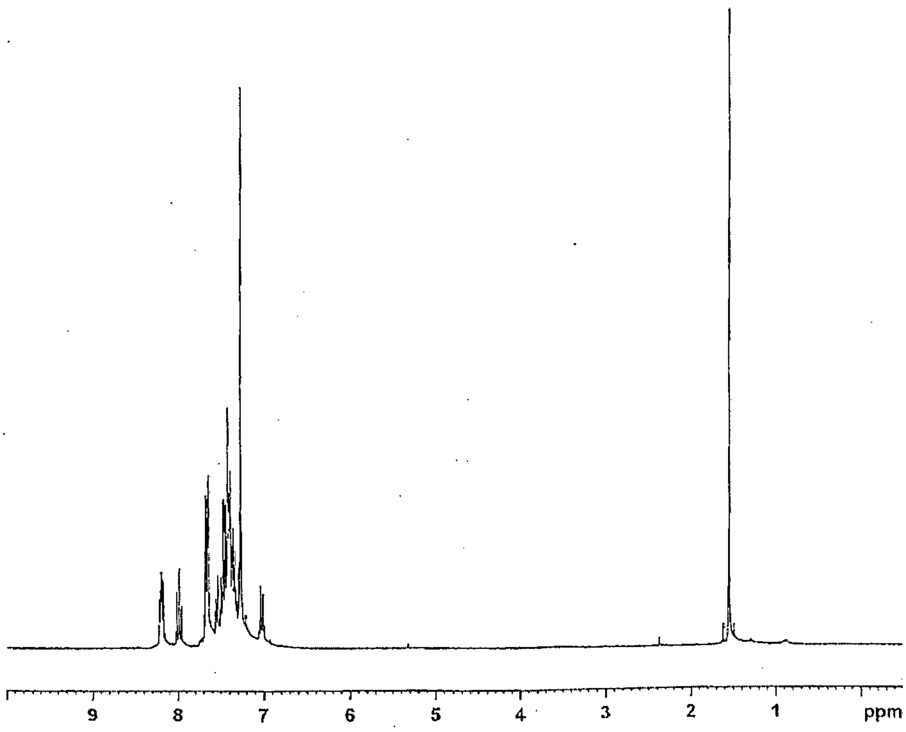

Fig. 7

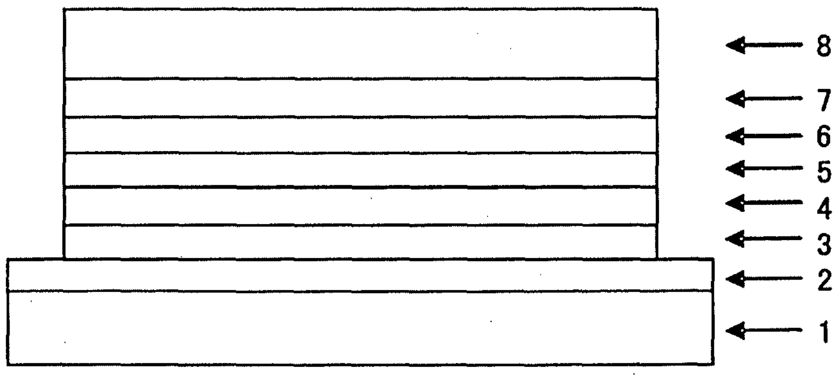


8: CATHODE
7: ELECTRON INJECTION LAYER
6: ELECTRON-TRANSPORTING LAYER
5: LUMINOUS LAYER
4: HOLE-TRANSPORTING LAYER
3: HOLE INJECTION LAYER
2: TRANSPARENT ANODE
1: GLASS SUBSTRATE

**INTERNATIONAL SEARCH REPORT**

International application No.
PCT/JP2014/065215

A. CLASSIFICATION OF SUBJECT MATTER
*C07D491/147*(2006.01)i, *C09K11/06*(2006.01)i, *H01L51/50*(2006.01)i

According to International Patent Classification (IPC) or to both national classification and IPC

B. FIELDS SEARCHED

Minimum documentation searched (classification system followed by classification symbols)
C07D491/147, C09K11/06, H01L51/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2011/125020 A1 (BASF (CHINA) CO., LTD.), 13 October 2011 (13.10.2011), claims 1, 9; pages 29 to 42, 93 to 96 & JP 2013-528929 A & EP 2556075 A1 | 1-13 |
| Y | WO 2011/099451 A1 (Nippon Steel Chemical Co., Ltd.), 18 August 2011 (18.08.2011), claims; paragraphs [0055] to [0059], [0075] to [0108] & US 2012/0305904 A1 & EP 2535958 A1 & WO 2011/099451 A1 | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 September, 2014 (02.09.14) | 09 September, 2014 (09.09.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2014/065215

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/107244 A2 (DOW ADVANCED DISPLAY MATERIALS, LTD.), 23 September 2010 (23.09.2010), claims & JP 2012-520872 A | 1-13 |
| A | WO 2012/169821 A1 (ROHM AND HAAS ELECTRONIC MATERIALS KOREA LTD.), 13 December 2012 (13.12.2012), claims & KR 10-2012-0136618 A & CN 103703003 A & TW 201305313 A | 1-13 |
| A | US 2012/0168734 A1 (PARK JUNGHWAN), 05 July 2012 (05.07.2012), claims & WO 2011/019173 A2 | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006151979 A
- WO 200862636 A


### Non-patent literature cited in the description


- *Synth. Commun.,* 1981, vol. 11, 513